# EUROPEAN PATENT APPLICATION

(11) **EP 4 759 922 A1**
(43) Date of publication of application: **17.06.2026**
(21) Application number: 24851868.0
(22) Date of filing: 07.08.2024
(51) Int. Cl.: C12N 1/21, C12N 1/15, C12N 1/19, C12N 5/10, C12N 15/09

(54) **METHOD FOR CONDITIONALLY INACTIVATING GENE, AND CELL THEREFOR**

(30) Priority: 07.08.2023 JP 2023128341
(71) Applicant: Logomix, Inc., Tokyo 113-0023 (JP)
(72) Inventor: AIZAWA, Yasunori, Tokyo 104-0053 (JP); OHNO, Tomoyuki, Tokyo 104-0053 (JP); KUDO, Kai, Tokyo 104-0053 (JP)
(74) Representative: Klöckner, Christoph
(86) International application number: PCT/JP2024/028181
(87) International publication number: WO 2025/033445

(57) **Abstract**

The present disclosure provides a method for destruction of a gene for inactivating a function of the one or more exogenous genes included in the cassette by inactivating a cassette including one or more exogenous genes complementing one or more destroyed endogenous genes, and a cell used for the method. The present disclosure also provides a method for inactivating a cassette that has become unnecessary when a time for which one or more exogenous genes included in the cassette are necessary for a cell has passed, and a cell used for the method.

## Description

### TECHNICAL FIELD

The present disclosure relates to a method for conditionally inactivating a gene, and a cell therefor. Specifically, the present disclosure relates to a method for destroying a gene, wherein a cassette containing one or more exogenous genes that complement one or more destroyed endogenous genes is inactivated, thereby inactivating said one or more exogenous genes contained in the cassette, and a cell used for the method. The present disclosure also relates to a method for inactivating a cassette that has become unnecessary when a cell has passed the time when one or more exogenous genes contained in the cassette are necessary, and a cell used for the method.

### BACKGROUND ART

Various methods for gene destruction have been developed. Systems in which gene destruction is induced only under specific conditions (i.e., conditional knockout systems) have also been established. For example, as disclosed in Patent Document 1, in a conditional knockout system, typically, a technique is known in which two site-specific recombination sequences (e.g., loxP sequences) are arranged so as to flank an endogenous gene, and a site-specific recombinase (e.g., Cre recombinase) is allowed to act thereon, thereby removing the endogenous gene from the genome. By allowing a site-specific recombinase to act on a genome at a desired time, an endogenous gene can be conditionally destroyed. In addition, by expressing a site-specific recombinase in a tissue-specific manner, an endogenous gene can be destroyed in a tissue-specific manner. In Non-Patent Document 1, an episomal vector is disclosed. In Non-Patent Documents 2 and 3, methods for removing an episomal vector such as a plasmid are disclosed. In Non-Patent Documents 4 and 5, and 6, a human artificial chromosome and a yeast artificial chromosome are disclosed. In Patent Document 2, a plasmid having a temperature-sensitive origin of replication is disclosed, and a technique for stopping replication of the plasmid by raising the temperature is disclosed. In Non-Patent Document 7, a technique for removing an artificial chromosome is disclosed. In Non-Patent Document 7, a technique for removing an artificial chromosome by a CRISPR/Cas system is disclosed.

### PRIOR ART DOCUMENTS

### PATENT DOCUMENTS

[Patent Document 1] US 4959317 A
[Patent Document 2] US 5616480 A

### NON-PATENT DOCUMENT

[Non-Patent Document 1] K Van Craenenbroeck et al., Eur J Biochem. 2000. 267(18):5665-78
[Non-Patent Document 2] Trevors, J.T., FEMS Microbiology Reviews. 1986, 32, 149-157
[Non-Patent Document 3] Lee, M. et al., Exp Mol Med. 2019 Jul; 51(7): 82
[Non-Patent Document 4] M, Ayabe et al., Biochemical and Biophysical Research Communications., 2004, 321(2):280-90
[Non-Patent Document 5] Harrington JJ et al., 1997, Nature Genetics.15(4):345-55
[Non-Patent Document 6] Murray, A. W., Szostak, J. W., Nature. 1983 Sep; 305(5931):189-93
[Non-Patent Document 7] Z Iida, Y. et al., DNA Res. 2010, 17(5):293-301
[Non-Patent Document 8] Zuo, E. et al., Genome Biol. 2017, 18(1):224

### SUMMARY OF THE INVENTION

The present disclosure provides a method for conditionally deleting a gene, and a cell therefor. Specifically, the present disclosure provides a cell having a cassette containing one or more exogenous genes that complement one or more destroyed endogenous genes, although the one or more endogenous genes are destroyed. This cell can exhibit no phenotype due to destruction because the exogenous gene complements the influence of the destroyed endogenous gene. By inactivating (e.g., removing) the cassette containing the exogenous gene from the cell, the one or more exogenous genes contained in the cassette are collectively inactivated (e.g., removed) at the timing of inactivating (e.g., removing) the cassette, whereby the influence of the destroyed endogenous gene can be exerted. Thus, the present disclosure provides a new conditional knockout technique by conditionally inactivating (e.g., removing) an exogenous gene that complements a destroyed endogenous gene. The present disclosure also provides a method for examining the function of a destroyed endogenous gene at a desired timing using the conditional knockout technique, and a cell used for the technique and method. The present disclosure further provides a method in which, while the cell maintains the cassette on DNA when one or more exogenous genes contained in the cassette are necessary, the cassette is inactivated (e.g., removed) when the one or more exogenous genes contained in the cassette become unnecessary, and a cell used for the method. In the present disclosure, a method involving or not involving destruction of an endogenous gene is also provided. In the present disclosure, a cell having a cassette containing one or more exogenous genes (e.g., knockdown nucleic acids) can be provided, and by inactivating (e.g., removing) the cassette, the one or more exogenous genes can be collectively removed from the cell or inactivated.

According to the present disclosure, for example, the following inventions are provided.
(1) A genetically modified cell, comprising: a cassette containing n or more exogenous genes encoding a physiologically functional product, wherein the exogenous genes are operably linked to one or more control sequences, the cell can express each of the exogenous genes,
   wherein the cassette is configured to be conditionally inactivated (e.g., removed) {for example, the cassette is located between two target sequences of a sequence-specific DNA cleavage enzyme or two recognition sequences of a site-specific recombinase, and inactivation may be performed by allowing a sequence-specific DNA cleavage enzyme or a site-specific recombinase to act on each of the two target sequences, or, when the cassette is present on extrachromosomal DNA, it may be performed by destroying a replication apparatus (e.g., a centromere or an origin of replication) of the extrachromosomal DNA},
   preferably, wherein endogenous genes corresponding to a part or all of the n or more exogenous genes are destroyed {in the above, n is a natural number of 1 or more. } .
(2) The cell according to (1) above, wherein n is 2 or more.
(3) The cell according to (1) or (2) above, wherein the destroyed endogenous gene includes a gene related to or involved in survival or proliferation of the cell, or a candidate gene thereof.
(4) The cell according to (1) or (2) above, wherein the destroyed endogenous gene includes a gene related to or involved in differentiation control of the cell or a candidate gene thereof.
(5) The cell according to (1) or (2) above, wherein the destroyed endogenous gene includes a gene related to or involved in a physiological function of the cell, or a candidate gene thereof.
(6) The cell according to any one of (1) to (5) above, wherein the cassette is located between two target sequences of a sequence-specific DNA cleavage enzyme or two recognition sequences of a site-specific recombinase, inactivation is performed by allowing a sequence-specific DNA cleavage enzyme or a site-specific recombinase to act on each of the two target sequences, and the control sequence is located outside a region between the two target sequences or the two recognition sequences (including the sequences).
(7) The cell according to any one of (1) to (6) above, wherein the cassette further carries a gene encoding a knockdown nucleic acid.
(8) A composition for use in producing the cell according to any one of (1) to (7) above, comprising a cell having an exogenous target region (also referred to as "landing pad") for integrating the cassette {the landing pad may be present on a genome or extrachromosomal DNA},
   wherein the exogenous target region comprises a target sequence of a sequence-specific DNA cleavage enzyme.
(9) The composition according to (8) above, wherein the exogenous target region comprises two alleles, each allele has a distinguishably different positive selection marker, and each allele further has a negative selection marker {however, when the positive selection marker also serves as the negative selection marker, the allele is not required to have a further negative marker.}.
(10) A method comprising:
   (A) culturing the cell according to any one of (1) to (7) above under appropriate conditions;
   (B) inactivating the cassette after culture to obtain a cell in which the cassette has been inactivated; and
   (C) further culturing under appropriate conditions the cell in which the cassette has been inactivated.
(11) The method according to (10) above, wherein the destroyed gene is a gene related to or involved in survival of the cell, a gene related to or involved in proliferation of the cell, or a candidate gene thereof, and the step (B) is performed after proliferating the cell in the step (A).
(12) The method according to (10) above, wherein the destroyed gene is a gene related to or involved in differentiation control of the cell or a candidate gene thereof, and the step (B) is performed after differentiating the cell in the step (A).
(13) The method according to (10) above, wherein the destroyed gene is a gene related to or involved in differentiation control of the cell or a candidate gene thereof, and the method comprises subjecting the cell to differentiation treatment in the culture of the step (C) after performing the step (B).
(14) The method according to (10) above, wherein the destroyed gene is a gene related to or involved in a physiological function of the cell, or a candidate gene thereof, and the step (B) is performed after proliferating the cell in the step (A).
(15) The cell according to any one of the above, wherein the cell has extrachromosomal DNA, and the cassette is contained in the extrachromosomal DNA {here, it is preferable that endogenous genes corresponding to a part or all of the n or more exogenous genes are destroyed}.
(16) The cell according to (15) above, wherein the cassette is located between two target sequences of a sequence-specific DNA cleavage enzyme or two recognition sequences of a site-specific recombinase, and inactivation is performed by allowing a sequence-specific DNA cleavage enzyme or a site-specific recombinase to act on each of the two target sequences.
(17) The cell according to (15) above, wherein the extrachromosomal DNA is an artificial chromosome, has alpha satellite DNA (or type I alphoid DNA) of a centromere region, the alpha satellite DNA has a tetracycline operator sequence (tet-O), and binding of a transcription silencer (e.g., tTS) induces heterochromatinization in the artificial chromosome, so that the extrachromosomal DNA is configured to drop out from the cell.
(18) The cell according to (15) above, wherein the extrachromosomal DNA is an episomal vector, and the episomal vector is configured to be conditionally inactivated {for example, the extrachromosomal DNA is cleaved and inactivated; the extrachromosomal DNA has an origin of replication, and the origin of replication is configured to be cleaved or dropped out from the episomal vector {for example, the origin of replication is located between two target sequences of a sequence-specific DNA cleavage enzyme or two recognition sequences of a site-specific recombinase, and dropping out is performed by allowing a sequence-specific DNA cleavage enzyme or a site-specific recombinase to act on each of the two target sequences}; or the episomal vector contains a drug resistance gene and is configured to drop out in the absence of a corresponding drug}.
(19) The invention according to any one of the above, wherein the cassette is contained in a genome of the cell.

(101) The cell according to any one of (1) to (7), (15), and (16) above, wherein the cassette contains a gene encoding a negative selection marker in addition to the n exogenous genes.

(102) The cell according to any one of (1) to (7), (15), and (16) above, wherein the cassette contains a gene encoding a selection marker (i.e., a positive selection marker) in addition to the n exogenous genes.

(103) The cell according to any one of (1) to (7), (15), and (16) above, wherein the cassette contains genes encoding a positive selection marker and a negative selection marker in addition to the n exogenous genes.

(104) The cell according to any one of (1) to (7), (15), and (16) above, wherein the cassette contains a gene encoding a marker that can be used for both positive selection and negative selection (e.g., a visualization marker) in addition to the n exogenous genes.

(201) A composition comprising a cell having an exogenous target region (also referred to as "landing pad") {the landing pad may be present on a genome or extrachromosomal DNA},
wherein the exogenous target region has a DNA fragment introduction site {for example, the exogenous target region contains two target sequences of a sequence-specific DNA cleavage enzyme or two recognition sequences of a site-specific recombinase for cassette removal, and has a DNA fragment introduction site between the two target sequences or the two recognition sequences}, the DNA fragment introduction site contains a target sequence of a sequence-specific DNA cleavage enzyme for DNA fragment introduction, and the two target sequences of the sequence-specific DNA cleavage enzyme for cassette removal are not cleaved by the sequence-specific DNA cleavage enzyme for DNA fragment introduction {wherein the DNA fragment contains n or more exogenous genes, and n is a natural number of 1 or more, preferably a natural number of 2 or more}.

(202) The cell according to (201) above, further having a control sequence.

(203) The cell according to (201) or (202) above, further having a control sequence between the two target sequences or the two recognition sequences.

(204) The cell according to (201) or (202) above, further having a control sequence outside a region between the two target sequences or the two recognition sequences.

(205) The cell according to any one of (201) to (204) above, having insulator sequences at both ends of a series of regions including the control sequence and a target sequence of a sequence-specific DNA cleavage enzyme for cassette introduction.

(206) The cell according to any one of (201) to (205) above, further having a negative selection marker in a region between the two target sequences of a sequence-specific DNA cleavage enzyme or the two recognition sequences of a site-specific recombinase for cassette removal.

(207) The cell according to any one of (201) to (205) above, having no negative selection marker in a region between the two target sequences of a sequence-specific DNA cleavage enzyme or the two recognition sequences of a site-specific recombinase for cassette removal.

(208) The cell according to any one of (201) to (205) above, wherein the exogenous target region has two or more alleles, and each allele has a distinguishably different positive selection marker.

(209) The cell according to (206) above, wherein the exogenous target region has two or more alleles, and each allele has a distinguishably different positive selection marker.

(210) The cell according to (207) above, wherein the exogenous target region has two or more alleles, and each allele has a distinguishably different positive selection marker.

(211) The cell according to any one of (201) to (210) above, wherein the cell has extrachromosomal DNA, and the exogenous target region is contained in the extrachromosomal DNA.

(212) The cell according to (211) above, wherein the cassette is located between two target sequences of a sequence-specific DNA cleavage enzyme or two recognition sequences of a site-specific recombinase, and inactivation is performed by allowing a sequence-specific DNA cleavage enzyme or a site-specific recombinase to act on each of the two target sequences.

(213) The cell according to (211) above, wherein the extrachromosomal DNA is an artificial chromosome, has alpha satellite DNA (or type I alphoid DNA) of a centromere region, the alpha satellite DNA has a tetracycline operator sequence (tet-O), and binding of a transcription silencer (e.g., tTS) induces heterochromatinization in the artificial chromosome, so that the extrachromosomal DNA is configured to drop out from the cell.

(214) The cell according to (211) above, wherein the extrachromosomal DNA is an episomal vector, and the episomal vector is configured to be conditionally inactivated {for example, it is cleaved and inactivated; it has an origin of replication, and the origin of replication is configured to be cleaved or dropped out from the plasmid {for example, the origin of replication is located between two target sequences of a sequence-specific DNA cleavage enzyme or two recognition sequences of a site-specific recombinase, and dropping out is performed by allowing a sequence-specific DNA cleavage enzyme or a site-specific recombinase to act on each of the two target sequences}; or it contains a drug resistance gene and is configured to drop out in the absence of a corresponding drug}.

(215) The invention according to any one of the above, wherein the cassette is contained in a genome of the cell.

(301) A method, comprising introducing n or more exogenous genes into a cleavage site by cleaving a target sequence of a sequence-specific DNA cleavage enzyme for DNA fragment introduction in the presence of a DNA fragment containing n or more exogenous genes in the cell according to any one of (201) to (210) above.

(401) A composition comprising a cell having an exogenous target region (also referred to as "landing pad"),
wherein the exogenous target region has a DNA fragment introduction site {for example, the exogenous target region contains two target sequences of a sequence-specific DNA cleavage enzyme or two recognition sequences of a site-specific recombinase for cassette removal, and has a DNA fragment introduction site between the two target sequences or the two recognition sequences}, the DNA fragment introduction site contains n or more exogenous genes, and the two target sequences of the sequence-specific DNA cleavage enzyme for cassette removal are not cleaved by the sequence-specific DNA cleavage enzyme for DNA fragment introduction, wherein the DNA fragment contains n or more exogenous genes.

(402) The cell according to (401) above, further having a control sequence.

(403) The cell according to (401) or (402) above, further having a control sequence between the two target sequences or the two recognition sequences.

(404) The cell according to (401) or (402) above, further having a control sequence outside a region between the two target sequences or the two recognition sequences.

(405) The cell according to any one of (402) to (405) above, having insulator sequences at both ends of a series of regions including the control sequence and a target sequence of a sequence-specific DNA cleavage enzyme for cassette introduction.

(406) The cell according to any one of (401) to (405) above, having a negative selection marker in a region between the two target sequences of a sequence-specific DNA cleavage enzyme or the two recognition sequences of a site-specific recombinase for cassette removal.

(407) The cell according to any one of (401) to (405) above, having no negative selection marker in a region between the two target sequences of a sequence-specific DNA cleavage enzyme or the two recognition sequences of a site-specific recombinase for cassette removal.

(408) The cell according to any one of (401) to (405) above, wherein the exogenous target region has two or more alleles, and each allele has a distinguishably different positive selection marker.

(409) The cell according to (406) above, wherein the exogenous target region has two or more alleles, and each allele has a distinguishably different positive selection marker.

(410) The cell according to (407) above, wherein the exogenous target region has two or more alleles, and each allele has a distinguishably different positive selection marker.

(411) The cell according to any one of (401) to (410) above, wherein the cell has extrachromosomal DNA, and the exogenous target region is contained in the extrachromosomal DNA.

(412) The cell according to (411) above, wherein the cassette is located between two target sequences of a sequence-specific DNA cleavage enzyme or two recognition sequences of a site-specific recombinase, and inactivation is performed by allowing a sequence-specific DNA cleavage enzyme or a site-specific recombinase to act on each of the two target sequences.

(413) The cell according to (411) above, wherein the extrachromosomal DNA is an artificial chromosome, has alpha satellite DNA (or type I alphoid DNA) of a centromere region, the alpha satellite DNA has a tetracycline operator sequence (tet-O), and binding of a transcription silencer (e.g., tTS) induces heterochromatinization in the artificial chromosome, so that the extrachromosomal DNA is configured to drop out from the cell.

(414) The cell according to (411) above, wherein the extrachromosomal DNA is an episomal vector, and the episomal vector is configured to be conditionally inactivated {for example, the extrachromosomal DNA is cleaved and inactivated; the episomal vector has an origin of replication, and the origin of replication is configured to be cleaved or dropped out from the episomal vector {for example, the origin of replication is located between two target sequences of a sequence-specific DNA cleavage enzyme or two recognition sequences of a site-specific recombinase, and dropping out is performed by allowing a sequence-specific DNA cleavage enzyme or a site-specific recombinase to act on each of the two target sequences}; or the episomal vector contains a drug resistance gene and is configured to drop out in the absence of a corresponding drug}.

(415) The invention according to any one of the above, wherein the cassette is contained in a genome of the cell.

(1001) A genetically modified cell,
comprising: a cassette containing n or more exogenous genes encoding a physiologically functional product, wherein the exogenous genes are operably linked to one or more control sequences, and the cell is capable of expressing each of the exogenous genes,
wherein the cassette is configured to be conditionally inactivated (e.g., removed) {for example, the cassette is located between two target sequences of a sequence-specific DNA cleavage enzyme or two recognition sequences of a site-specific recombinase, and inactivation may be performed by allowing a sequence-specific DNA cleavage enzyme or a site-specific recombinase to act on each of the two target sequences, or, when the cassette is present on extrachromosomal DNA, the inactivation may be performed by destruction of the extrachromosomal DNA or by destroying a replication apparatus (e.g., a centromere or an origin of replication) thereof},
preferably, wherein endogenous genes corresponding to a part or all of the n or more exogenous genes are destroyed {in the above, n is a natural number of 1 or more. } .

(1002) The cell according to (1001) above, wherein n is 2 or more.

(1003) The cell according to (1001) or (1002) above, wherein the n or more exogenous genes include genes encoding m or more knockdown nucleic acids {in the above, m is a natural number of 1 or more or 2 or more, and n or less.}.

(1004) The cell according to any one of (1001) to (1003) above, wherein the m or more knockdown nucleic acids include one or more knockdown nucleic acids selected from the group consisting of siRNA, shRNA, miRNA, and antisense oligos.

(1005) The cell according to any one of (1001) to (1004) above, wherein the n or more exogenous genes include p or more genes having a physiological function (e.g., may be selected from the group consisting of a gene related to or involved in survival of the cell, a gene related to or involved in proliferation of the cell, a gene related to or involved in differentiation control of the cell, and a gene related to or involved in a physiological function of the cell) {in the above, p is a natural number of 1 or more or 2 or more, and a natural number of n or less, the n or more exogenous genes may or may not include a knockdown nucleic acid, and when the n or more exogenous genes include m or more knockdown nucleic acids, m is a natural number of 1 or more or 2 or more, and the sum of p and m is n or less.}.

(1006) The cell according to any one of (1001) to (1005) above, wherein the control sequence is located outside a region between the two target sequences or the two recognition sequences (including the target sequences or the recognition sequences).

(1007) A composition for use in producing the cell according to any one of (1001) to (1006) above, comprising a cell having an exogenous target region (also referred to as "landing pad") for integrating the cassette, wherein the exogenous target region comprises a target sequence of a sequence-specific DNA cleavage enzyme.

(1008) The composition according to (7) above, wherein the exogenous target region comprises two alleles, each allele has a distinguishably different positive selection marker, and each allele further has a negative selection marker {however, when the positive selection marker also serves as the negative selection marker, the allele is not required to have a further negative marker.}.

(1009) A method, comprising:
(A) culturing the cell according to any one of (1001) to (1006) above under appropriate conditions;
(B) inactivating (e.g., removing) the cassette after culture to obtain a cell in which the cassette has been inactivated (e.g., removed); and
(C) further culturing the cell in which the cassette has been inactivated (e.g., removed) under appropriate conditions.

(1010) The method according to (1009) above, wherein the destroyed gene is a gene related to or involved in survival of the cell, a gene related to or involved in proliferation of the cell, or a candidate gene thereof, and the step (B) is performed after proliferating the cell in the step (A).

(1011) The method according to (1009) above, wherein the destroyed gene is a gene related to or involved in differentiation control of the cell or a candidate gene thereof, and the step (B) is performed after differentiating the cell in the step (A).

(1012) The method according to (1009) above, wherein the destroyed gene is a gene related to or involved in differentiation control of the cell or a candidate gene thereof, and the method comprises subjecting the cell to differentiation treatment in the culture of the step (C) after performing the step (B).

(1013) The method according to (1009) above, wherein the destroyed gene is a gene related to or involved in a physiological function of the cell (e.g., a gene related to or involved in metabolism of the cell, e.g., a gene encoding a metabolic enzyme of the cell) or a candidate gene thereof, and the step (B) is performed after proliferating the cell in the step (A).

(1101) The invention according to any one of the above, wherein the cell is a human cell.

(1102) The invention according to any one of the above, wherein the cell is a non-human cell.

(1103) The invention according to any one of the above, wherein the cell is a cell of a microorganism.

(1104) The cell according to any one of the above, wherein the cell has extrachromosomal DNA, and the cassette is contained in the extrachromosomal DNA {here, it is preferable that endogenous genes corresponding to a part or all of the n or more exogenous genes are destroyed}.

(1105) The cell according to (1104) above, wherein the cassette is located between two target sequences of a sequence-specific DNA cleavage enzyme or two recognition sequences of a site-specific recombinase, and inactivation is performed by allowing a sequence-specific DNA cleavage enzyme or a site-specific recombinase to act on each of the two target sequences.

(1106) The cell according to (1104) above, wherein the extrachromosomal DNA is an artificial chromosome, has alpha satellite DNA (or type I alphoid DNA) of a centromere region, the alpha satellite DNA has a tetracycline operator sequence (tet-O), and binding of a transcription silencer (e.g., tTS) induces heterochromatinization in the artificial chromosome, so that the extrachromosomal DNA is configured to drop out from the cell.

(1107) The cell according to (1104) above, wherein the extrachromosomal DNA is an episomal vector, and the episomal vector is configured to be conditionally inactivated {for example, the episomal vector is cleaved and inactivated; the episomal vector has an origin of replication, and the origin of replication is configured to be cleaved or dropped out from the episomal vector {for example, the origin of replication is located between two target sequences of a sequence-specific DNA cleavage enzyme or two recognition sequences of a site-specific recombinase, and dropping out is performed by allowing a sequence-specific DNA cleavage enzyme or a site-specific recombinase to act on each of the two target sequences}; or the episomal vector contains a drug resistance gene and is configured to drop out in the absence of a corresponding drug}.

(1108) The invention according to any one of the above, wherein the cassette is contained in a genome of the cell.

(1201) A genetically modified cell, having extrachromosomal DNA, wherein the extrachromosomal DNA contains n or more exogenous genes encoding a physiologically functional product, the exogenous genes are operably linked to one or more control sequences, and the cell is capable of expressing each of the exogenous genes, wherein the cassette is configured to be conditionally inactivated (e.g., removed) {for example, the cassette is located between two target sequences of a sequence-specific DNA cleavage enzyme or two recognition sequences of a site-specific recombinase, and inactivation may be performed by allowing a sequence-specific DNA cleavage enzyme or a site-specific recombinase to act on each of the two target sequences, or, when the cassette is present on extrachromosomal DNA, it may be performed by destruction of the extrachromosomal DNA or by destroying a replication apparatus (e.g., a centromere or an origin of replication) thereof}, preferably, a cell wherein endogenous genes corresponding to a part or all of the n or more exogenous genes are destroyed {in the above, n is a natural number of 1 or more.}.

(1202) The cell according to (1201) above, wherein n is 2 or more.

(1203) The cell according to (1201) or (1202) above, wherein the destroyed endogenous gene includes a gene related to or involved in survival of the cell, a gene related to or involved in proliferation of the cell, or a candidate gene thereof.

(1204) The cell according to (1201) or (1202) above, wherein the destroyed endogenous gene includes a gene related to or involved in differentiation control of the cell or a candidate gene thereof.

(1205) The cell according to (1201) or (1202) above, wherein the destroyed endogenous gene includes a gene related to or involved in a physiological function of the cell, or a candidate gene thereof.

(1206) The cell according to any one of (1201) to (1205) above, wherein the control sequence is located outside a region between the two target sequences or the two recognition sequences (including the sequences).

(1207) The cell according to any one of (1201) to (1206) above, wherein the cassette further carries a gene encoding a knockdown nucleic acid.

(1208) A composition for use in producing the cell according to any one of (1201) to (1207) above, comprising a cell having an exogenous target region (also referred to as "landing pad") for integrating the cassette,
wherein the exogenous target region comprises a target sequence of a sequence-specific DNA cleavage enzyme.

(1209) The composition according to (1208) above, wherein the exogenous target region comprises two alleles, each allele has a distinguishably different positive selection marker, and each allele further has a negative selection marker {however, when the positive selection marker also serves as the negative selection marker, it does not have to have a further negative marker. } .

(1210) A method comprising: (A) culturing the cell according to any one of (1201) to (1207) above under appropriate conditions; (B) inactivating (e.g., removing) the cassette after culture to obtain a cell in which the cassette has been inactivated (e.g., removed); and (C) further culturing the cell in which the cassette has been inactivated (e.g., removed) under appropriate conditions.

(1211) The method according to (1210) above, wherein the destroyed gene is a gene related to or involved in survival of the cell, a gene related to or involved in proliferation of the cell, or a candidate gene thereof, and the step (B) is performed after proliferating the cell in the step (A).

(1212) The method according to (1210) above, wherein the destroyed gene is a gene related to or involved in differentiation control of the cell or a candidate gene thereof, and the step (B) is performed after differentiating the cell in the step (A).

(1213) The method according to (1210) above, wherein the destroyed gene is a gene related to or involved in differentiation control of the cell or a candidate gene thereof, and the method comprises subjecting the cell to differentiation treatment in the culture of the step (C) after performing the step (B).

(1214) The method according to (1210) above, wherein the destroyed gene is a gene related to or involved in a physiological function of the cell, or a candidate gene thereof, and the step (B) is performed after proliferating the cell in the step (A).

(1215) The cell according to (1214) above, wherein the cassette is located between two target sequences of a sequence-specific DNA cleavage enzyme or two recognition sequences of a site-specific recombinase, and inactivation is performed by allowing a sequence-specific DNA cleavage enzyme or a site-specific recombinase to act on each of the two target sequences.

(1216) The cell according to (1214) above, wherein the extrachromosomal DNA is an artificial chromosome, has alpha satellite DNA (or type I alphoid DNA) of a centromere region, the alpha satellite DNA has a tetracycline operator sequence (tet-O), and binding of a transcription silencer (e.g., tTS) induces heterochromatinization in the artificial chromosome, so that the extrachromosomal DNA is configured to drop out from the cell.

(1217) The cell according to (1214) above, wherein the extrachromosomal DNA is an episomal vector, and the episomal vector is configured to be conditionally inactivated {for example, it is cleaved and inactivated; it has an origin of replication, and the origin of replication is configured to be cleaved or dropped out from the episomal vector {for example, the origin of replication is located between two target sequences of a sequence-specific DNA cleavage enzyme or two recognition sequences of a site-specific recombinase, and dropping out is performed by allowing a sequence-specific DNA cleavage enzyme or a site-specific recombinase to act on each of the two target sequences}; or it contains a drug resistance gene and is configured to drop out in the absence of a corresponding drug}.

(1301) A genetically modified cell, having extrachromosomal DNA, wherein the extrachromosomal DNA contains n or more exogenous genes encoding a physiologically functional product, the exogenous genes are operably linked to one or more control sequences, and the cell is capable of expressing each of the exogenous genes, wherein the extrachromosomal DNA is configured to be conditionally inactivated {in the above, n is a natural number of 1 or more. }.

(1302) The cell according to (1301) above, wherein n is 2 or more.

(1303) The cell according to (1301) or (1302) above, wherein the n or more exogenous genes include genes encoding m or more knockdown nucleic acids {in the above, m is a natural number of 1 or more or 2 or more, and n or less.}.

(1304) The cell according to any one of (1301) to (1303) above, wherein the m or more knockdown nucleic acids include one or more knockdown nucleic acids selected from the group consisting of siRNA, shRNA, miRNA, and antisense oligos.

(1305) The cell according to any one of (1301) to (1304) above, wherein the n or more exogenous genes include p or more genes having a physiological function (e.g., may be selected from the group consisting of a gene related to or involved in survival of the cell, a gene related to or involved in proliferation of the cell, a gene related to or involved in differentiation control of the cell, and a gene related to or involved in a physiological function of the cell) {in the above, p is a natural number of 1 or more or 2 or more, and a natural number of n or less, the n or more exogenous genes may or may not include a knockdown nucleic acid, and when the n or more exogenous genes include m or more knockdown nucleic acids, m is a natural number of 1 or more or 2 or more, and the sum of p and m is n or less.}.

(1306) The cell according to any one of (1301) to (1305) above, wherein the control sequence is located outside a region between the two target sequences or the two recognition sequences (including the target sequences or the recognition sequences).

(1307) A composition for use in producing the cell according to any one of (1301) to (1306) above, comprising a cell having an exogenous target region (also referred to as "landing pad") for integrating the cassette, wherein the exogenous target region comprises a target sequence of a sequence-specific DNA cleavage enzyme.

(1308) The composition according to claim 7, wherein the exogenous target region comprises two alleles, each allele has a distinguishably different positive selection marker, and each allele further has a negative selection marker {however, when the positive selection marker also serves as the negative selection marker, it does not have to have a further negative marker.}.

(1309) A method comprising: (A) culturing the cell according to any one of (1301) to (1306) above under appropriate conditions; (B) inactivating (e.g., removing) the cassette after culture to obtain a cell in which the cassette has been inactivated (e.g., removed); and
(C) further culturing the cell in which the cassette has been inactivated (e.g., removed) under appropriate conditions.

(1310) The method according to (1309) above, wherein the destroyed gene is a gene related to or involved in survival of the cell, a gene related to or involved in proliferation of the cell, or a candidate gene thereof, and the step (B) is performed after proliferating the cell in the step (A).

(1311) The method according to (1309) above, wherein the destroyed gene is a gene related to or involved in differentiation control of the cell or a candidate gene thereof, and the step (B) is performed after differentiating the cell in the step (A).

(1312) The method according to (1309) above, wherein the destroyed gene is a gene related to or involved in differentiation control of the cell or a candidate gene thereof, and the method comprises subjecting the cell to differentiation treatment in the culture of the step (C) after performing the step (B).

(1313) The method according to (1309) above, wherein the destroyed gene is a gene related to or involved in a physiological function of the cell (e.g., a gene related to metabolism of the cell, e.g., a gene encoding a metabolic enzyme of the cell) or a candidate gene thereof, and the step (B) is performed after proliferating the cell in the step (A).

(1310) The cell according to (1310) above, wherein the cassette is located between two target sequences of a sequence-specific DNA cleavage enzyme or two recognition sequences of a site-specific recombinase, and inactivation is performed by allowing a sequence-specific DNA cleavage enzyme or a site-specific recombinase to act on each of the two target sequences.

(1311) The cell according to (1310) above, wherein the extrachromosomal DNA is an artificial chromosome, has alpha satellite DNA (or type I alphoid DNA) of a centromere region, the alpha satellite DNA has a tetracycline operator sequence (tet-O), and binding of a transcription silencer (e.g., tTS) induces heterochromatinization in the artificial chromosome, so that the extrachromosomal DNA is configured to drop out from the cell.

(1312) The cell according to (1310) above, wherein the extrachromosomal DNA is an episomal vector, and the episomal vector is configured to be conditionally inactivated {for example, it is cleaved and inactivated; it has an origin of replication, and the origin of replication is configured to be cleaved or dropped out from the episomal vector {for example, the origin of replication is located between two target sequences of a sequence-specific DNA cleavage enzyme or two recognition sequences of a site-specific recombinase, and dropping out is performed by allowing a sequence-specific DNA cleavage enzyme or a site-specific recombinase to act on each of the two target sequences}; or it contains a drug resistance gene and is configured to drop out in the absence of a corresponding drug}.

(1400) The cell according to any one of the above, wherein the extrachromosomal DNA is an artificial chromosome.

(1401) The cell according to any one of the above, wherein the extrachromosomal DNA is a human artificial chromosome.

(1402) The cell according to any one of the above, wherein the extrachromosomal DNA is a yeast artificial chromosome.

(1403) The cell according to any one of the above, wherein the extrachromosomal DNA is a bacterial artificial chromosome.

(1404) The cell according to any one of the above, wherein the extrachromosomal DNA is a P1 bacteriophage artificial chromosome.

(1405) The cell according to any one of the above, wherein the extrachromosomal DNA is an episomal vector.

(1406) The cell according to any one of the above, wherein the extrachromosomal DNA is a BK virus-derived episomal vector.

(1407) The cell according to any one of the above, wherein the extrachromosomal DNA is a bovine papillomavirus 1-derived episomal vector.

(1408) The cell according to any one of the above, wherein the extrachromosomal DNA is an Epstein-Barr virus-derived episomal vector.

(1409) The cell according to any one of the above, wherein the extrachromosomal DNA is a Sendai virus vector. (1410) The cell according to any one of the above, wherein the extrachromosomal DNA is plasmid DNA.

(1501) The cell according to any one of (1400) to (1409) above, wherein the extrachromosomal DNA has a suicide gene operably linked to an inducible promoter.

(1502) The cell according to any one of (1400) to (1409) above, wherein the extrachromosomal DNA has a drug resistance gene operably linked to a promoter.

(1503) The cell according to any one of the above, wherein the extrachromosomal DNA has two or more recognition sequences of a site-specific recombinase and one or two or more target sequences of a sequence-specific DNA recombination enzyme, and is configured such that the extrachromosomal DNA is destroyed by allowing the site-specific recombinase or the sequence-specific DNA recombination enzyme to act on the extrachromosomal DNA.

(1504) The cell according to any one of the above, wherein the extrachromosomal DNA has temperature sensitivity and is configured to be removed from the cell by raising the temperature.

(1505) The cell according to any one of the above, wherein the extrachromosomal DNA is a temperature-sensitive Sendai virus vector, and is removed from the cell by temperature sensitivity.

(1506) The cell according to any one of the above, wherein the extrachromosomal DNA is, the cell has extrachromosomal DNA, the cassette is contained in the extrachromosomal DNA, and one or more, or preferably two or more, of endogenous genes corresponding to a part or all of the exogenous genes contained in the cassette are destroyed.

(1507) The cell according to (1400) above, wherein the cassette is contained in the extrachromosomal DNA, and one or more, or preferably two or more, of endogenous genes corresponding to a part or all of the exogenous genes contained in the cassette are destroyed.

(1508) The cell according to (1401) above, wherein the cassette is contained in the extrachromosomal DNA, and one or more, or preferably two or more, of endogenous genes corresponding to a part or all of the exogenous genes contained in the cassette are destroyed.

(1509) The cell according to (1402) above, wherein the cassette is contained in the extrachromosomal DNA, and one or more, or preferably two or more, of endogenous genes corresponding to a part or all of the exogenous genes contained in the cassette are destroyed.

(1510) The cell according to (1403) above, wherein the cassette is contained in the extrachromosomal DNA, and one or more, or preferably two or more, of endogenous genes corresponding to a part or all of the exogenous genes contained in the cassette are destroyed.

(1511) The cell according to (1404) above, wherein the cassette is contained in the extrachromosomal DNA, and one or more, or preferably two or more, of endogenous genes corresponding to a part or all of the exogenous genes contained in the cassette are destroyed.

(1512) The cell according to (1405) above, wherein the cassette is contained in the extrachromosomal DNA, and one or more, or preferably two or more, of endogenous genes corresponding to a part or all of the exogenous genes contained in the cassette are destroyed.

(1513) The cell according to (1406) above, wherein the cassette is contained in the extrachromosomal DNA, and one or more, or preferably two or more, of endogenous genes corresponding to a part or all of the exogenous genes contained in the cassette are destroyed.

(1514) The cell according to (1407) above, wherein the cassette is contained in the extrachromosomal DNA, and one or more, or preferably two or more, of endogenous genes corresponding to a part or all of the exogenous genes contained in the cassette are destroyed.

(1515) The cell according to (1408) above, wherein the cassette is contained in the extrachromosomal DNA, and one or more, or preferably two or more, of endogenous genes corresponding to a part or all of the exogenous genes contained in the cassette are destroyed.

(1516) The cell according to (1409) above, wherein the cassette is contained in the extrachromosomal DNA, and one or more, or preferably two or more, of endogenous genes corresponding to a part or all of the exogenous genes contained in the cassette are destroyed.

(1517) The cell according to (1410) above, wherein the cassette is contained in the extrachromosomal DNA, and one or more, or preferably two or more, of endogenous genes corresponding to a part or all of the exogenous genes contained in the cassette are destroyed.

(1518) The cell according to (1501) above, wherein the cassette is contained in the extrachromosomal DNA, and one or more, or preferably two or more, of endogenous genes corresponding to a part or all of the exogenous genes contained in the cassette are destroyed.

(1519) The cell according to (1502) above, wherein the cassette is contained in the extrachromosomal DNA, and one or more, or preferably two or more, of endogenous genes corresponding to a part or all of the exogenous genes contained in the cassette are destroyed.

(1520) The cell according to (1503) above, wherein the cassette is contained in the extrachromosomal DNA, and one or more, or preferably two or more, of endogenous genes corresponding to a part or all of the exogenous genes contained in the cassette are destroyed.

(1521) The cell according to (1504) above, wherein the cassette is contained in the extrachromosomal DNA, and one or more, or preferably two or more, of endogenous genes corresponding to a part or all of the exogenous genes contained in the cassette are destroyed.

(1522) The cell according to (1505) above, wherein the cassette is contained in the extrachromosomal DNA, and one or more, or preferably two or more, of endogenous genes corresponding to a part or all of the exogenous genes contained in the cassette are destroyed.

### [Brief Description of the Drawings]

[FIG. 1A] A technique is shown in which genes A, B, C, and D are ectopically introduced into DNA having a landing pad, then endogenous A, B, C, and D are destroyed to collect genes A to D in one place, and the collected genes A to D are conditionally knocked out. In FIG. 1, as an example, for conditional knockout, loxP, which is a recognition sequence of a site-specific recombinase, sandwiches a cassette containing genes A to D, and the cassette is removed from DNA by inducing Cre recombinase and allowing it to act on the DNA.

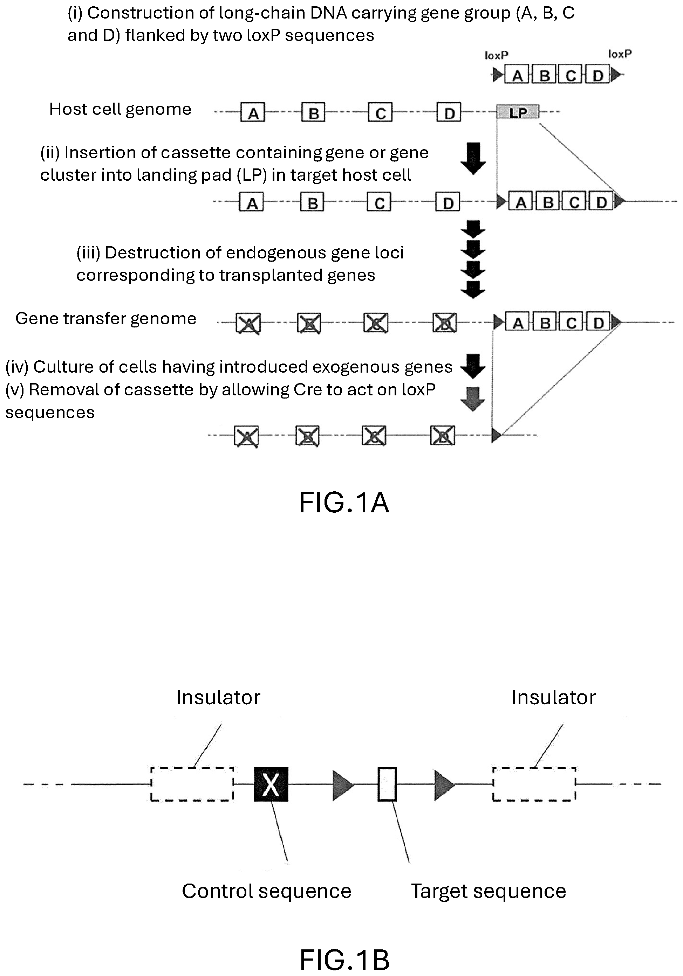

[FIG. 1B] Shows an example of the configuration of a landing pad. In the figure, triangles are, as an example, recognition sequences of a site-specific recombinase. A cassette is a region between two recognition sequences of a site-specific recombinase, and in this example, a control sequence is arranged outside the cassette. A target sequence of a sequence-specific DNA cleavage enzyme exists inside the cassette, and a fragment containing n or more exogenous genes is inserted at the location of the target sequence. In the figure, triangles are recognition sequences of a site-specific recombinase. Note that the insulator does not have to be present.

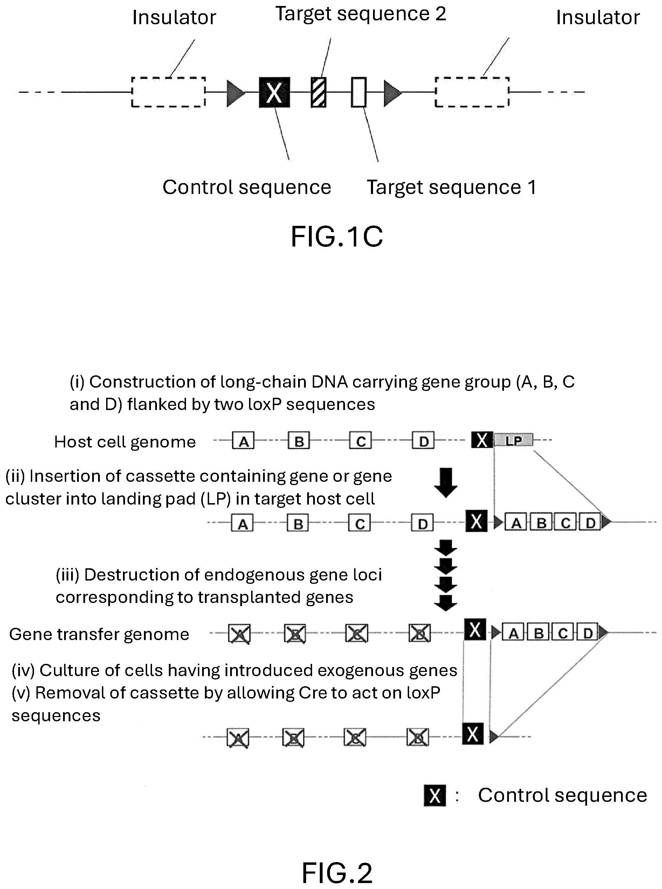

[FIG. 1C] Shows an example of the configuration of a landing pad. In the figure, triangles are recognition sequences of a site-specific recombinase. A cassette is a region between two recognition sequences of a site-specific recombinase, and in this example, a control sequence is arranged inside the cassette. A target sequence 1 of a sequence-specific DNA cleavage enzyme exists inside the cassette, and a fragment containing n or more exogenous genes is inserted at the location of the target sequence 1. A target sequence 2 of a sequence-specific DNA cleavage enzyme exists inside the cassette, and after cassette removal, the target sequence 2 is cleaved, and the cassette is destroyed. Note that the insulator does not have to be present.
[FIG. 2] Illustrates an example in which a control sequence exists outside the cassette. In this example, after cassette removal, the removed cassette will be dissociated from the control sequence.
[FIG. 3] Illustrates an example in which a control sequence exists inside the cassette. In this example, after cassette removal, the removed cassette accompanies the control sequence.
[FIG. 4A] Illustrates an example in which a control sequence exists inside the cassette. The cassette has a target sequence of a sequence-specific DNA cleavage enzyme inside.
[FIG. 4B] Shows a scheme in which a sequence-specific DNA cleavage enzyme is allowed to act on the target sequence of the removed cassette to destroy the cassette. The cleaved DNA is removed by an intracellular mechanism.

### [Description of the Embodiments]

### <Definitions of Terms>

In the present specification, terms in the singular are used in a sense that does not exclude the plural.

In the present specification, "comprising" means that an unspecified third component may be included. In the present specification, "consisting of" means that an unspecified third component is not substantially included. "Not substantially included" does not exclude inclusion of a third component to an extent that cannot be removed in a manufacturing process or below a detection limit. In the present specification, "consisting only of" means that an unspecified third component is not included.

In the present specification, a "cell" is a structural and functional basic unit possessed by all living organisms. A cell has a closed space covered with a cell membrane, and has genomic DNA inside thereof. A cell may be a prokaryotic cell (prokaryote) and a eukaryotic cell (eukaryote). A cell may be a cell of a unicellular organism or a cell of a multicellular organism. A cell may be a singularized cell. A cell may be an isolated cell. In certain embodiments, the cell may be a cell selected from the group consisting of pluripotent cells, and pluripotent stem cells (such as embryonic stem cells and induced pluripotent stem cells). In certain embodiments, the pluripotent cell and the pluripotent stem cell are of a naive type. In certain embodiments, the pluripotent cell and the pluripotent stem cell may be of a primed type. In certain embodiments, the cell may be an epiblast stem cell, or is not an epiblast stem cell. In certain embodiments, the cell may be a tissue stem cell. In certain embodiments, the cell may be a somatic cell. In certain embodiments, the cell may be a germline cell (e.g., a germ cell, a gamete, e.g., an oocyte, an ovum, a spermatogonium, etc.). In certain embodiments, the cell may be a cell line. In certain embodiments, the cell may be an immortalized cell. In certain embodiments, the cell may be a cancer cell. In certain embodiments, the cell may be a non-cancer cell. In certain embodiments, the cell may be a cell of a diseased patient. In certain embodiments, the cell may be a cell of a healthy subject. In certain embodiments, the cell may be a cell selected from the group consisting of animal cells (e.g., human cells), e.g., insect cells (e.g., silkworm cells), HEK293 cells, HEK293T cells, Expi293F (trademark) cells, FreeStyle (trademark) 293F cells, Chinese hamster ovary cells (CHO cells), CHO-S cells, CHO-K1 cells, and ExpiCHO cells, and derivative cells from these cells. In certain embodiments, the cell may be a cell of a non-human organism. In certain embodiments, the non-human organism may be is selected from the group consisting of a yeast (e.g., fission yeast or budding yeast, e.g., Saccharomyces cerevisiae, Saccharomyces carlsbergensis, Saccharomyces fragilis, Saccharomyces rouxii, etc., genus Candida such as Candida utilis, Candida tropicalis, genus Pichia, genus Kluyveromyces, genus Yarrowia, genus Hansenula, genus Endomyces, etc. In certain embodiments, the non-human organism may be a filamentous fungus (e.g., Aspergillus, Trichoderma, Humicola, Acremonium, Fusarium, and Penicillium species). In certain embodiments, the non-human organism is Bacillus subtilis. In certain embodiments, the non-human organism is a hydrogen bacterium. A hydrogen bacterium is a bacterium having an ability to oxidize hydrogen and performing carbon assimilation using energy generated by the oxidation reaction (also referred to as "hydrogen-oxidizing bacterium"). Hydrogen bacteria (e.g., aerobic hydrogen bacteria, Knallgas bacteria) can use hydrogen as an electron donor and oxygen as an electron acceptor, and have an ability to reductively assimilate carbon dioxide gas to synthesize organic matter. Examples of hydrogen bacteria include, but are not limited to, bacteria of the genus Caminibacter, bacteria of the genus Aquifex, bacteria of the genus Ralstonia, and bacteria of the genus Paracoccus. Examples of hydrogen bacteria also include, for example, bacteria of the genus Alcaligenes, bacteria of the genus Pseudomonas, bacteria of the genus Bacillus, and bacteria of the genus Hydrogenobacter. Hydrogen bacteria can grow well in the presence of sufficient nutrients, hydrogen, oxygen, and carbon dioxide. Since hydrogen bacteria produce substances such as proteins from carbon dioxide, they have a carbon dioxide reduction effect, and can produce organic matter such as proteins (e.g., animal proteins), foods, plastics, chemicals, biofuels, etc., so their utilization is expected. Hydrogen bacteria preferably have a group 1h/5 hydrogenase. In certain preferred embodiments, the hydrogen bacterium may be Ralstonia eutropha.

In the present specification, a "control sequence" refers to a sequence that promotes transcription from DNA to RNA (particularly mRNA). Examples of control sequences include promoters. In the present specification, a "promoter" refers to a portion that exists near (particularly upstream of) a DNA region to be expressed and has a function of promoting transcription from the DNA to RNA (particularly mRNA). The promoter is not particularly limited, and for example, various pol II promoters can be used. Examples of pol II promoters include, but are not limited to, CMV promoter, EF1 promoter (EF1alpha promoter), SV40 promoter, MSCV promoter, hTERT promoter, beta-actin promoter, CAG promoter, CBh promoter, and the like. The promoter may be an inducible promoter. An inducible promoter is a promoter capable of inducing expression of a polynucleotide functionally linked to the promoter only in the presence of an inducer that drives the promoter. Examples of inducible promoters include promoters that induce gene expression by heating, such as heat shock promoters. In addition, inducible promoters include promoters in which the inducer driving the promoter is a drug. Examples of such drug-inducible promoters include Cumate operator sequences, lambda operator sequences (e.g., 12 x lambda Op), tetracycline-based inducible promoters, and the like. Examples of tetracycline-based inducible promoters include promoters that drive gene expression in the presence of tetracycline or a derivative thereof (e.g., doxycycline), or reverse tetracycline-controlled transactivator (rtTA). Examples of tetracycline-based inducible promoters include TRE3G promoters.

In the present specification, "operably linking" means linking a gene (DNA) to a control sequence (e.g., a promoter) so that expression is driven. By operably linking a gene to a control sequence, expression of the gene can be induced by the action of the control sequence.

In the present specification, a "gene" refers to a polynucleotide containing at least one open reading frame encoding a specific protein, or containing a frame encoding noncoding RNA. The gene preferably encodes a protein and may contain both exons and introns. The gene preferably encodes a protein and may contain only exons. The gene preferably has a sequence of complementary DNA (cDNA) of RNA (e.g., mRNA) generated by transcription from the gene.

The term "allele" refers to a set of sequences present at the same locus on a chromosomal genome. In certain embodiments, two alleles exist at the same locus in a diploid cell, and three alleles exist at the same locus in a triploid cell. Also, in certain embodiments, additional alleles may be formed by abnormal copies of chromosomes or abnormal additional copies of the locus.

The terms "genome modification" or "genome editing" are used interchangeably and refer to inducing a mutation at a desired position (target region) on a genome. Genome modification may involve the use of a sequence-specific nucleic acid cleavage molecule designed to cleave target region DNA. In a preferred embodiment, genome modification may involve the use of a nuclease engineered to cleave DNA of a target region. In a preferred embodiment, genome modification may involve the use of a nuclease (e.g., TALEN or ZFN) engineered to cleave a target sequence having a specific base sequence in a target region. In a preferred embodiment, in genome modification, there may be cases where a sequence-specific endonuclease such as a meganuclease, which is a restriction enzyme having only one cleavage site in the genome (e.g., a restriction enzyme having 16-base sequence specificity (theoretically present at a rate of one in 4¹⁶ bases), a restriction enzyme having 17-base sequence specificity (theoretically present at a rate of one in 4¹⁷ bases), and a restriction enzyme having 18-base sequence specificity (theoretically present at a rate of one in 4¹⁸ bases)), can be used to cleave a target sequence having a specific base sequence in a target region. Typically, the use of a site-specific nuclease induces a double-strand break (DSB) in the DNA of the target region, after which the genome is repaired by endogenous processes of the cell, such as Homology-Directed Repair (HDR) and Non-Homologous End-Joining Repair (NHEJ). NHEJ is a repair method that joins double-strand broken ends without using donor DNA, and insertion and/or deletion (indel) are frequently induced during repair. HDR is a repair mechanism using donor DNA, and it is also possible to introduce a desired mutation into a target region. As a genome modification technique, for example, a CRISPR/Cas system is preferably exemplified. Examples of meganucleases include I-SceI, I-SceII, I-SceIII, I-SceIV, I-SceV, I-SceVI, I-SceVII, I-CeuI, I-CeuAIIP, I-CreI, I-CrepsbIP, I-CrepsbIIP, I-CrepsbIIIP, I-CrepsbIVP, I-TliI, I-PpoI, PI-PspI, F-SceI, F-SceII, F-SuvI, F-TevI, F-TevII, I-Amal, I-Anil, I-Chul, I-CmoeI, I-Cpal, I-CpaII, I-CsmI, I-Cvul, I-CvuAIP, I-DdiI, I-DdiII, I-DirI, I-DmoI, I-HmuI, I-HmuII, I-HsNIP, I-L1aI, I-MsoI, I-NaaI, I-NanI, I-NclIP, I-NgrIP, I-NitI, I-NjaI, I-Nsp236IP, I-PakI, I-PboIP, I-PcuIP, I-PcuAI, I-PcuVI, I-PgrIP, I-PobIP, I-PorI, I-PorIIP, I-PbpIP, I-SpBetaIP, I-ScaI, I-SexIP, I-SneIP, I-SpomI, I-SpomCP, I-SpomIP, I-SpomIIP, I-SquIP, I-Ssp68031, I-SthPhiJP, I-SthPhiST3P, I-SthPhiSTe3bP, I-TdeIP, I-TevI, I-TevII, I-TevIII, I-UarAP, I-UarHGPAIP, I-UarHGPA13P, I-VinIP, I-ZbiIP, PI-Mtul, PI-MtuHIP PI-MtuHIIP, PI-Pful, PI-PfuII, PI-PkoI, PI-PkoII, PI-Rma43812IP, PI-SpBetaIP, PI-SceI, PI-TfuI, PI-TfuII, PI-ThyI, PI-TIiI, and PI-TliII, and meganucleases selected from the group consisting of functional derivative restriction enzymes thereof and cleavage sites (or recognition sites) thereof, preferably meganucleases which are restriction enzymes having sequence specificity of 18 bases or more and cleavage sites (or recognition sites) thereof, and particularly meganucleases that do not cleave the genome of a cell at one or more locations and cleavage sites thereof can be used. Genome modification can also be used for modification of extrachromosomal DNA.

The term "target region" refers to a DNA region targeted by a sequence-specific DNA cleavage enzyme. A sequence-specific DNA cleavage enzyme typically cleaves DNA within or near a target region.

The term "corresponding" in the context referring to a gene means being the same gene or a functional counterpart. The same gene refers to a gene present at the same locus in the same animal species, and does not exclude sequence differences, but can exert the same physiological function. Therefore, an endogenous gene corresponding to an exogenous gene means an endogenous gene recognized as the same gene as the exogenous gene, although sequence differences are not excluded. A functional counterpart means a gene having an equivalent function.

The term "donor DNA" refers to DNA used for repair of a double-strand break of DNA, which is capable of homologous recombination with DNA around a target region. The donor DNA contains an upstream base sequence and a downstream base sequence of the target region (e.g., base sequences adjacent to the target region) as homology arms. In the present specification, a homology arm consisting of a base sequence on the upstream side (e.g., a base sequence adjacent to the upstream side) of the target region may be described as an "upstream homology arm", and a homology arm consisting of a base sequence on the downstream side (e.g., a base sequence adjacent to the downstream side) of the target sequence may be described as a "downstream homology arm". The donor DNA can contain a desired base sequence between the upstream homology arm and the downstream homology arm. The length of each homology arm is preferably 300 bp or more, usually about 500 to 3000 bp. The lengths of the upstream homology arm and the downstream homology arm may be the same or different from each other. If induction of homologous recombination between the target region and the donor DNA is successful after sequence-dependent cleavage, the sequence between the upstream base sequence and the downstream base sequence of the target region is replaced with the sequence of the donor DNA.

"Upstream" of a target region means a DNA region located on the 5' side of a reference nucleotide strand in double-stranded DNA of the target region. "Downstream" of a target region means DNA located on the 3' side of a reference nucleotide strand. When the target region contains a protein coding sequence, the reference nucleotide strand is usually a sense strand. Generally, a promoter is located upstream of a protein coding sequence. A terminator is located downstream of a protein coding sequence.

The term "sequence-specific nucleic acid cleavage molecule" refers to a molecule capable of recognizing a specific nucleic acid sequence and cleaving a nucleic acid at the specific nucleic acid sequence. A sequence-specific nucleic acid cleavage molecule is a molecule having an activity of cleaving a nucleic acid in a sequence-specific manner (sequence-specific nucleic acid cleavage activity).

The term "target sequence" refers to a DNA sequence in DNA that is a target for cleavage by a sequence-specific nucleic acid cleavage molecule. When the sequence-specific nucleic acid cleavage molecule is a Cas protein, the target sequence refers to a DNA sequence that is a target for cleavage by the Cas protein. When a Cas9 protein is used as the Cas protein, the target sequence needs to be a sequence adjacent to the 5' side of a protospacer adjacent motif (PAM). As the target sequence, a sequence of 17 to 30 bases (preferably 18 to 25 bases, more preferably 19 to 22 bases, still more preferably 20 bases) adjacent immediately before the 5' side of PAM is usually selected. For designing a target sequence, known design tools such as CRISPR DESIGN (crispr.mit.edu/) can be used. The target sequence is preferably a unique sequence on DNA (particularly, genome) from the viewpoint of reducing off-target cleavage.

In the present specification, "under appropriate conditions" refers to conditions under which an object can be achieved. For example, conditions suitable for culture refer to conditions suitable for maintenance and/or proliferation of cells. Conditions suitable for differentiation may be conditions under which differentiation factors necessary for cells to differentiate into specific cells are sufficiently supplied in addition to maintenance of cells.

In the present specification, a "cassette" means a unit of nucleic acid capable of inactivation, or a unit of nucleic acid capable of removal from other parts. Elements contained in a cassette can be integrally inactivated (e.g., removed) by a single operation.

In the present specification, "conditional" means being triggered by specific conditions. Conditional knockout means knockout triggered by specific conditions. Inactivating conditionally means inactivation triggered by specific conditions. Specific conditions may be satisfied by applying a specific stimulus to a cell. Inactivation of a cassette means inactivating the function of an exogenous gene in the cassette by, for example, destruction (e.g., cleavage), inhibition, or removal, etc. of the cassette; destruction (e.g., cleavage), inhibition, or removal, etc. of a promoter; or destruction (e.g., cleavage), inhibition, or removal, etc. of a functional linkage between a gene and a promoter; or, although possible when the cassette is contained in extrachromosomal DNA, eliminating the entire extrachromosomal DNA containing the cassette to be inactivated. In certain preferred embodiments, inactivation may be removal of a cassette or extrachromosomal DNA.

Conditional inactivation can be achieved by conditional knockout. Conditional knockout can be caused, for example, by the presence, expression, or addition of a site-specific recombinase in a cell, or by the presence, expression, or addition of a sequence-specific DNA cleavage enzyme. The presence, expression, or addition of a site-specific recombinase in a cell, or the presence, expression, or addition of a sequence-specific DNA cleavage enzyme can occur by driving an inducible control sequence by operably linking these enzymes to the inducible control sequence and allowing them to exist in the cell. Driving of the inducible control sequence can occur by contacting a factor that drives the inducible control sequence with the cell. Examples of site-specific recombinases include Cre recombinase, Flp recombinase, Dre recombinase, phage integrase, and various site-specific recombinases derived therefrom. Examples of site-specific recombinases also include transposases possessed by DNA transposons. Cre recombinase recognizes loxP sequences and drops a region sandwiched between two loxP sequences from DNA. Flp recombinase recognizes FRT sequences and drops a region sandwiched between two FRT sequences from DNA. Dre recombinase drops a region sandwiched between two Dre sequences from DNA. Phage integrase drops a region sandwiched between attL and attR from DNA with the aid of an excision factor (Xis). Transposase recognizes inverted terminal repeat sequences (ITRs) respectively possessed at the ends of the genome of a transposon, and drops a region sandwiched between two ITRs from DNA. Methods for introducing a desired cassette (or gene) into DNA or dropping a cassette from DNA using a piggyBac vector (or piggyBac transposon vector) are well known to those skilled in the art. Examples of phage integrases include tyrosine type integrases such as lambda phage integrase and HK022 bacteriophage integrase, and serine type integrases such as phiC31 integrase and TG1 integrase. Sequences derived from loxP sequences include lox511, lox2272, and loxFAS recognized by Cre recombinase, but these sequences do not recombine with loxP.
Those skilled in the art can appropriately select a site-specific recombinase and its recognition sequence and use them for removing a cassette. The recognition sequence of a site-specific recombinase may be selected from the group consisting of loxP, lox511, lox2272, lox66, lox71, loxM2, lox5171, FRT, FRT11, FRT71, attL, attR, and Dre sites. Recombinases that induce recombination in these recognition sequences are well known. The two recognition sequences are oriented and arranged on DNA so that the sandwiched region is removed. Those skilled in the art can determine appropriate conditions for operating the above recombinase, and under the appropriate conditions, can inhibit or stop the expression of the function of the exogenous gene in the cassette by removing the cassette and/or the control sequence between the recognition sequences.

Conditional inactivation can also be achieved by transcription suppression techniques such as CRISPRi. In CRISPRi, a transcription repression domain is linked to a dCas9 protein that has lost endonuclease activity (a mutant in which amino acid mutations have been introduced into the active center of DNA cleavage to inhibit nuclease activity, e.g., a mutant of Cas9 of Streptococcus pyogenes having D10A and H840A (Cas9 of Streptococcus pyogenes has, for example, Cas9 of UniProt accession number Q99ZW2)), and the transcription repression domain is recruited onto DNA in a target sequence-specific manner, whereby transcription from a target control sequence can be suppressed. The transcription suppression ability is strong, and for example, expression can be reduced to one several-hundredth. Examples of transcription repression domains include KRAB domain (Kruppel-associated box domain) and transcription repression (TRD) domain of MeCP2, and fusion proteins thereof. Examples of transcription repression domains include peptides selected from the group consisting of KRAB, DMNT1, SET1, HDAC1, DMNT3A, SETD8, EXH2, SUV39H1, PHF19, SALI, NUE, SUR4, KYP, DIM5, HDAC8, SIRT3, SIRT6, MESOLO4, SET8, HST2, COBB, NCOR, MeCP2, SIN3A, HDT1, MBD2B, NIPP1, and HP1A, or transcription repression domains thereof, or fusion proteins of two peptides or transcription repression (TRD) domains thereof (see Nat Methods. 2018 Aug; 15(8): 611-616). CRISPRi can inactivate a cassette, for example, by targeting a control sequence or an exogenous gene. Such CRISPRi can be achieved using a guide RNA having a sequence targeting a control sequence or an exogenous gene (e.g., single-stranded guide RNA or crRNA) as a guide RNA.

In order to perform inactivation conditionally, proteins for inactivation such as site-specific recombinases, sequence-specific DNA cleavage enzymes, and dCAS9 can be linked to a tissue-specific promoter or an inducible promoter. Tissue-specific promoters and inducible promoters are frequently used in the field of conditional knockout, and those skilled in the art can appropriately select a tissue-specific promoter or an inducible promoter to achieve conditional cassette inactivation. Inducible promoters may be as described above.

### <Cell of the Present Disclosure>

According to the present disclosure, a genetically modified cell is provided. According to the present disclosure, the cell has a cassette containing n or more exogenous genes, the exogenous genes are operably linked to one or more control sequences, and the cell is capable of expressing each of the exogenous genes {where n is a natural number of 1 or more, or 2 or more. } .

According to certain embodiments of the present disclosure, the cell or the cassette is configured such that the cassette can be conditionally inactivated. Inactivation can be achieved by destruction (e.g., cleavage), inhibition, or removal, etc. of the cassette.

According to certain embodiments (first aspect) of the present disclosure, in the cell, the n or more exogenous genes include p or more genes encoding a physiologically functional product {where p is a natural number of 1 or more or 2 or more, and is n or a natural number less than n.}. It is possible to introduce n or more exogenous genes into a cell, induce a phenotype or observe a phenotype, and then inactivate (e.g., remove) the cassette. Also, certain embodiments (first aspect) of the present disclosure can be used to produce cells of the second aspect described below. Cells of the first aspect survive and preferably proliferate in culture under appropriate conditions.

According to certain embodiments (second aspect) of the present disclosure, in the cell, the n or more exogenous genes include p or more genes encoding a physiologically functional product {where p is a natural number of 1 or more or 2 or more, and is n or a natural number less than n.}, and endogenous genes corresponding to a part (e.g., 1 or more, 2 or more, 3 or more, 4 or more, 5 or more, 10 or more, or 20 or more) or all of the n or more exogenous genes are destroyed. Alternatively, in the cell, the n or more exogenous genes include p or more genes encoding a physiologically functional product {where p is a natural number of 1 or more or 2 or more, and is n or a natural number less than n.}, and endogenous genes corresponding to a part or all of the p or more genes encoding a physiologically functional product are destroyed. In this aspect, the exogenous gene encoding a physiologically functional product can complement the destroyed endogenous gene (e.g., its function). Let q be the number of genes whose functions are complemented by exogenous genes among destroyed endogenous genes {q is a natural number of 1 or more or preferably 2 or more, and is p or a natural number less than p.}. By inactivation (e.g., removal) of the cassette described later, it is expected that the effect of destruction of q endogenous genes appears in the cell. The cell of the second aspect survives and preferably proliferates in culture under appropriate conditions at least before inactivation (e.g., removal) of the cassette. The cell of the second aspect, after cassette inactivation (e.g., removal), for example, (i) survives and does not proliferate, (ii) survives and proliferates, or (iii) does not survive.

In certain embodiments, n and p are equal. In certain embodiments, n and p are different. Also, in certain embodiments, p and q are equal. In certain embodiments, p and q are different. In certain embodiments, n and p are equal, and p and q are equal. In certain embodiments, n and p are equal, and p and q are different. In certain embodiments, n and p are different, and p and q are equal. In certain embodiments, n and p are different, and p and q are different.

In the second aspect, the exogenous gene introduced into the cassette includes a gene capable of complementing the endogenous gene to be destroyed or at least its physiological function, and preferably includes the same gene as the endogenous gene. Thereby, even after the endogenous gene is destroyed, the exogenous gene introduced into the cassette can complement the endogenous gene to ensure cell functions as much as necessary. It is not necessarily required that all destroyed endogenous genes are complemented by exogenous genes. This is because it is not necessary to supply the same gene exogenously to the cell as long as necessary functions are retained even if destroyed. Also, the cassette may contain a gene different from the endogenous gene to be destroyed. A gene in the cassette different from the destroyed endogenous gene can confer a new function on the cell. The same gene is a gene present at a corresponding locus (i.e., the same locus) in the same biological species. Since the endogenous gene and the exogenous gene are the same gene, the function of the destroyed endogenous gene is complemented by the exogenous gene. By inactivation (e.g., removal) of the cassette, it is expected that the effect of destruction of q endogenous genes appears in the cell.

According to certain embodiments (third aspect) of the present disclosure, in the cell, the n or more exogenous genes include m or more knockdown nucleic acids {where m is a natural number of 1 or more or 2 or more.}. A knockdown nucleic acid is a nucleic acid that inhibits expression of one or more genes (preferably a single gene) in a nucleic acid (e.g., genome, etc.) present in a cell. As the knockdown nucleic acid, a knockout type nucleic acid consisting of RNA is advantageously used, and for example, may be selected from the group consisting of siRNA, shRNA, miRNA, and antisense oligos. In the third aspect, the endogenous gene may be destroyed or may not be destroyed, but the important point is that by inactivating (e.g., removing) the cassette, the gene encoding the knockdown nucleic acid is inactivated (e.g., removed), and expression of the knockdown nucleic acid can be reduced or eliminated. When a plurality of knockdown nucleic acids are contained in the cassette, expression of the plurality of knockdown nucleic acids can be collectively reduced or eliminated. Thereby, by conditionally inactivating (e.g., removing) the cassette, the knockdown effect can be released. When a plurality of knockdown nucleic acids are contained in the cassette, by inactivating (e.g., removing) the cassette at a desired timing, the knockdown effect by the plurality of knockdown nucleic acids can be released by this single operation. In certain embodiments, n and m are equal. In certain embodiments, n and m are different. The cassette can be inactivated (e.g., removed) at the timing of expression of a sequence-specific DNA cleavage enzyme or expression of a site-specific recombinase for inactivating (e.g., removing) the cassette. The enzyme can be expressed by driving an inducible promoter operably linked to a gene encoding the enzyme. Alternatively, inactivation (e.g., removal) of the cassette is also possible by introducing a sequence-specific DNA cleavage enzyme or a site-specific recombinase into the cell. In this way, when necessary, the cassette can be inactivated (e.g., removed) by allowing a sequence-specific DNA cleavage enzyme or a site-specific recombinase to act on the cassette. The cell of the third aspect survives and preferably proliferates in culture under appropriate conditions at least before inactivation (e.g., removal) of the cassette. The cell of the third aspect, after cassette inactivation (e.g., removal), (i) survives and does not proliferate, (ii) survives and proliferates, or (iii) does not survive.

siRNA is also referred to as small interfering RNA, and is a molecule capable of silencing expression of RNA having a homologous sequence (target RNA). The siRNA is not particularly limited as long as it can silence target RNA, but typically is a double-stranded RNA comprising a 20-25 mer RNA (guide strand) having a sequence complementary to target RNA and a 20-25 mer complementary strand thereof (passenger strand). In siRNA, the guide strand and the passenger strand hybridize to form double-stranded RNA. siRNA may have a sequence (overhang) that does not form a double strand at the 5' end or 3' end. The overhang may be, for example, about 1 to 3 mer. shRNA is also referred to as small hairpin RNA, and typically is a single-stranded RNA formed by linking two RNA strands in siRNA by a hairpin, and under physiological conditions, a guide strand portion and a passenger strand portion contained in shRNA can hybridize. shRNA is advantageous for expression in cells. This is because it is a single-stranded RNA, can be transcribed from one gene encoding shRNA, and the transcribed shRNA can form a functional form having a hairpin structure in the cell. miRNA is mature microRNA, is a single-stranded RNA of about 21 to 25, and is a molecule capable of suppressing expression of RNA having a complementary sequence. siRNA, shRNA, and miRNA are considered to be incorporated into a protein complex called RNA-induced silencing complex (RISC) to silence target RNA. Antisense oligos are single-stranded nucleic acids having a sequence complementary to target RNA. Antisense oligos can be single-stranded RNA, and the optimal length is usually considered to be 12 mer to 28 mer. It is said that antisense oligos of 12 mer or less increase the risk of non-specific silencing, and antisense oligos with a length exceeding 25 mer have reduced uptake efficiency into cells. For example, the length of the antisense oligo can be 18 mer to 30 mer, or 20 mer to 25 mer. In the present disclosure, antisense oligos can be generated by being transcribed from DNA in cells, and therefore longer antisense oligos can also be used. Those skilled in the art can appropriately determine the sequence and length of these knockdown nucleic acids considering their target specificity and expression efficiency. Knockdown nucleic acids are typically operably linked to a control sequence of RNA polymerase III (e.g., U6 promoter, T7 promoter, etc.).

The first to third aspects of the cell are not necessarily exclusive and can be compatible. In a fourth aspect, in the first to third aspects, n or more exogenous genes may be such that p of them are genes encoding physiologically functional products, and m of them are knockout type nucleic acids {where p and m are independently natural numbers of 1 or more, preferably 2 or more, and the sum of p and m is a natural number of n or less} . Also in the fourth aspect, endogenous genes corresponding to a part or all of the genes encoding physiologically functional products may be destroyed. In certain embodiments, p and q are equal. In certain embodiments, p and q are different. In a preferred embodiment, the sum of p and m can be a natural number of 2 or more, preferably 3 or more, more preferably 4 or more. The cell of the fourth aspect survives and preferably proliferates in culture under appropriate conditions at least before inactivation (e.g., removal) of the cassette. The cell of the fourth aspect, after cassette inactivation (e.g., removal), (i) survives and does not proliferate, (ii) survives and proliferates, or (iii) does not survive.

The cassette contains n or more exogenous genes. Here, n may be a natural number of 1 or more, preferably 2 or more, 3 or more, 4 or more, 5 or more, 6 or more, 7 or more, 8 or more, 9 or more, or 10 or more, but may be, for example, a natural number of 50 or less, 40 or less, 30 or less, 20 or less, 15 or less or 10 or less. n may be, for example, a natural number of 1 to 50, 1 to 40, 1 to 30, 1 to 20 or 1 to 10, 2 to 50, 2 to 40, 2 to 30, 2 to 20 or 2 to 10. The cell may contain two or more cassettes. The two cassettes contain a total of n or more exogenous genes, wherein n may be a natural number of 1 or more, preferably 2 or more, 3 or more, 4 or more, 5 or more, 6 or more, 7 or more, 8 or more, 9 or more, or 10 or more, but may be, for example, a natural number of 50 or less, 40 or less, 30 or less, 20 or less, 15 or less or 10 or less. n may be, for example, a natural number of 1 to 50, 1 to 40, 1 to 30, 1 to 20 or 1 to 10, 2 to 50, 2 to 40, 2 to 30, 2 to 20 or 2 to 10. Two or more cassettes can be removed independently, consecutively, sequentially, or simultaneously or together.

In the second aspect, the influence of destruction of the endogenous gene can be reduced by destroying the endogenous gene and supplying the same exogenous gene as the destroyed gene into the cassette, but the exogenous gene can be collectively inactivated (e.g., knocked out) by a single operation of inactivating (e.g., removing) the cassette, which is useful in that the effect of reducing the influence of destruction of the endogenous gene can be eliminated. In order to inactivate (e.g., destroy) a plurality of genes that originally existed apart on a nucleic acid (particularly genome) of a cell, it is necessary to inactivate (e.g., destroy) them individually, and it is not easy to inactivate (e.g., destroy) a plurality of genes from nucleic acid (particularly genome) at once. However, according to the present disclosure, the cell or the cassette is configured such that the entire cassette can be inactivated. According to the present disclosure, for example, it can be removed from a nucleic acid possessed by a cell (e.g., cell, or genome or extrachromosomal DNA). In addition, by removing the cassette from a nucleic acid possessed by a cell or from a cell, the n or more exogenous genes can be collectively inactivated (e.g., knocked out). In a cell in which the cassette has been inactivated (e.g., removed), the endogenous gene is destroyed, and after cassette inactivation (e.g., removal) (particularly, triggered by cassette inactivation (e.g., removal)), a phenotype due to destruction is expressed. This is the same in the fourth aspect.

In the third aspect, the function of knockdown nucleic acids contained in the cassette can be collectively stopped by inactivating (e.g., removing) the cassette, and the knockdown effect of the cell can be released at the timing of inactivating (e.g., removing) the cassette. This is the same in the fourth aspect.

The cassette carries an exogenous gene desired to be conditionally inactivated (e.g., deleted). The cassette may have a further natural sequence or artificial sequence in addition to the exogenous gene. Examples of the further natural sequence or artificial sequence include marker genes and other tool genes, and sequences for promoting expression of exogenous genes in the cassette or suppressing attenuation thereof. Here, "tool gene" is one that can be used as a tool for enhancing convenience of gene modification technology, such as detecting success or failure of gene modification or obtaining only cells that have been successfully modified. Sequences for promoting expression of exogenous genes in the cassette or suppressing attenuation thereof include control sequences, polyadenylation sequences, and insulators. Marker genes can be used to confirm introduction of the cassette onto DNA, or marker genes can be used to confirm removal of the cassette from DNA. The cassette may further contain a gene encoding a site-specific recombinase. r tool genes may be contained in the cassette {r is a natural number of 1 or more, and is a natural number that does not exceed n even when added to the number of the exogenous genes.}. Exogenous genes may also include such tool genes. r may be a natural number of 1 or more, 2 or more, 3 or more, 4 or more, 5 or more, 6 or more, 7 or more, 8 or more, 9 or more, 10 or more, and may be a natural number of 1 to 20 or 1 to 10.

The cassette may be present on the genome of the cell or may be present on DNA outside the genome (or outside the host chromosome). Therefore, inactivation (e.g., removal) of the cassette can be achieved by removal from the genome and removal from extrachromosomal DNA of the host, and removal of extrachromosomal DNA of the host. The cassette does not necessarily need to be removed from the cell as long as expression is inhibited, but in certain embodiments, removal of the cassette is removal from the cell. When the cassette is present on the genome, it is not particularly limited, but for example, it is located or integrated at a transcriptionally active locus or a safe harbor region on the genome. Here, "host" means that the cassette is derived from a cell into which it is introduced or said cell. The insertion position of the cassette may be, for example, ROSA26 locus. The insertion position of the cassette may be, for example, CH25h locus. The insertion position of the cassette may be, for example, AAVS1 locus. Even other than the safe harbor region, the cassette can be integrated at any location as long as it does not cause significant damage unacceptable to cell function due to destruction of existing genes or the like, or significantly inhibit expression of the cassette. When the cassette is present extrachromosomally in the host, one or more or preferably two or more endogenous genes corresponding to a part or all of exogenous genes contained in the cassette are genetically engineered (preferably destroyed). In certain preferred embodiments, the cassette is present extrachromosomally in the host, and two or more endogenous genes corresponding to a part or all of exogenous genes are destroyed.

The cassette may be inserted on DNA outside the genome (or outside the host chromosome), and is not particularly limited, but for example, may be contained on extrachromosomal DNA such as artificial chromosomes (e.g., artificial chromosomes for eukaryotic cells such as human artificial chromosomes (HAC), yeast artificial chromosomes (YAC), and artificial chromosomes for bacteria such as bacterial artificial chromosomes (BAC), and P1 bacteriophage artificial chromosomes (PAC), etc.) and episomal vectors (e.g., circular DNA such as plasmids and viral vectors, etc.). HAC is DNA created by including telomere and centromere regions and deleting other regions unnecessary for chromosome maintenance. HAC includes, but is not particularly limited to, for example, mini-chromosome types created by inserting telomere sequences into natural chromosomes to shorten chromosomes, and those created by integrating centromeres, telomeres, and various genes into linear YAC. YAC has a centromere, a telomere, a gene encoding a selection marker, and an autonomously replicating sequence (ARS). By having a centromere region, an artificial chromosome can be replicated in a cell and maintained in divided cells. BAC has a replicon of F factor and can be maintained in a single copy in a host cell (e.g., E. coli). PAC is a plasmid utilizing a replication system of P1 bacteriophage, and can be maintained in a host cell (e.g., E. coli) with a low copy number. These artificial chromosomes for bacteria can be removed from host cells by techniques similar to plasmids. It is preferable that these artificial chromosomes each have a cloning site. The cloning site has, for example, a restriction enzyme cleavage site, and is configured so that foreign DNA can be inserted. Artificial chromosomes are replicated and maintained independently without being inserted into host chromosomes, and when genes are loaded, they do not interfere with induction of expression of the genes. Artificial chromosomes may further contain selection marker genes. Episomal vectors are vectors capable of replicating outside host chromosomes. Episomal vectors include DNA vectors and RNA vectors; representative examples of DNA vectors include circular DNA such as plasmids, and representative examples of RNA vectors include mononegaviruses such as Sendai virus vectors. Plasmids are circular DNAs containing an origin of replication necessary for replication in host cells, and selection marker genes. Plasmids usually have a drug resistance gene and an origin of replication, and can be maintained in cells in the presence of drugs. Viral vectors are not particularly limited as long as they are not inserted into the genome and are maintained episomally, but examples thereof include Sendai virus vectors. Sendai virus is a virus having a single-stranded non-segmented negative-strand RNA genome classified into the family Paramyxoviridae. Sendai virus deficient in F protein necessary for infection is used as a vector, and F protein is supplied from packaging cells at the time of production, but after cell infection, infectious virus is not produced because there is no supply of F protein.

Inactivation (e.g., removal) of a cassette is also possible by removing the entire extrachromosomal DNA. For example, by destroying an origin of replication or a centromere of extrachromosomal DNA, by culturing in the absence of a drug corresponding to a loaded drug resistance gene, or by cleaving chromosomal DNA, extrachromosomal DNA can be removed from a host cell. Destruction of an origin of replication or a centromere can be performed by DNA cleavage with a site-specific recombinase or a sequence-specific DNA cleavage enzyme. In episomal vectors, the origin of replication is essential for replication, and destruction of the origin of replication can remove the episomal vector. Destruction of the origin of replication can be done, for example, by removal, cleavage, etc. of the origin of replication. In artificial chromosomes, the centromere is essential for replication, and destruction or removal of the centromere can remove the artificial chromosome. Destruction of the centromere can be done, for example, by removal of the centromere, promotion of heterochromatinization of the centromere, and the like. In certain embodiments, extrachromosomal DNA contains, for example, a recognition sequence of a sequence-specific DNA cleavage enzyme for cleavage, is cleaved by the sequence-specific DNA cleavage enzyme, and the extrachromosomal DNA is destroyed and disappears. In certain embodiments, extrachromosomal DNA has, for example, two recognition sequences of a site-specific recombinase or target sequences of a sequence-specific DNA cleavage enzyme sandwiching an origin of replication or a centromere, and a region containing the origin of replication or the centromere can be removed from the extrachromosomal DNA by the site-specific recombinase or the sequence-specific DNA cleavage enzyme. In certain embodiments, extrachromosomal DNA has one or more or a plurality of target sequences of a sequence-specific DNA cleavage enzyme within an origin of replication or a centromere, is cleaved by the sequence-specific DNA cleavage enzyme, and the extrachromosomal DNA is destroyed and disappears. As the sequence-specific DNA cleavage enzyme, a CRISPR/Cas system or a meganuclease can be used. Bacterial artificial chromosomes can be removed from inside host cells utilizing plasmid incompatibility. Bacterial artificial chromosomes can also be removed by one or more chemicals (e.g., plasmid curing agents) selected from the group consisting of acriflavine, acridine orange, ethidium bromide, quinacrine, coumermycin, novobiocin, mitomycin C, rifampicin, and sodium dodecyl sulfate (SDS) (Trevors, J.T., FEMS Microbiology Reviews. 1986, 32, 149-157). Examples of episomal vectors include BK virus-derived episomal vectors, bovine papillomavirus 1-derived episomal vectors, and Epstein-Barr virus-derived episomal vectors (K Van Craenenbroeck, P Vanhoenacker, G Haegeman , Eur J Biochem. 2000 Sep;267(18):5665-78). BK virus-derived episomal vectors typically include BKV early promoter (BKVE), BKori, BKV small T antigen (BKt), BKV large T antigen (BKT), and may additionally include genes encoding drug selection markers in E. coli and origins of replication, and genes encoding drug selection markers in eukaryotic cells. Bovine papillomavirus 1-derived episomal vectors (BPV-1 derived vectors) include BPV-1ori, and E1 to E8, and may additionally include genes encoding drug selection markers in E. coli and origins of replication, and genes encoding drug selection markers in eukaryotic cells. Epstein-Barr virus (EBV)-derived episomal vectors include EBNA1 and OriP, and may additionally include genes encoding drug selection markers in E. coli and origins of replication, and genes encoding drug selection markers in eukaryotic cells. Techniques for removing extrachromosomal DNA include, for example, a method of integrating a suicide gene, performing selection, and selecting only cells lacking extrachromosomal DNA in the division process (Lee, M. et al., Exp Mol Med. 2019 Jul; 51(7): 82), a method of inhibiting replication of an artificial chromosome by inhibiting the centromere of an artificial chromosome for eukaryotic cells to obtain cells that have lost the artificial chromosome (Iida, Y. et al., DNA Res. 2010 Oct;17(5):293-301), and applying a method of cleaving extrachromosomal DNA at one or more locations, preferably a plurality of locations, by a CRISPR/Cas system or a meganuclease to remove it from the cell (Zuo, E. et al., Genome Biol. 2017 Nov 24;18(1):224) to extrachromosomal DNA. A plasmid removal technique by temperature sensitivity (US 5616480 A) may be applied to extrachromosomal DNA. Those skilled in the art will be able to appropriately remove extrachromosomal DNA from cells. Temperature-sensitive Sendai virus vectors have temperature-sensitive mutations and can be removed from inside cells by raising the temperature (WO2023/127871A). Various temperature-sensitive mutations are known, and those skilled in the art can appropriately select and use them. Sendai virus vectors have a constituent protein (e.g., P protein) linked to a degron, and can be removed from inside cells by allowing a proteolytic accelerator to act thereon (WO2023/127871A).

Heterochromatinization of a centromere can be achieved by incorporating a target sequence of a transcription silencer into the centromere. For example, heterochromatinization of a centromere can be achieved by introducing a tetracycline operator sequence (tet-O) into the centromere and contacting a transcription silencer. As the transcription silencer, a fusion protein (tTS) of Tet repressor protein (TetR) and KRAB silencing domain (SDKid-1) can be used. When the transcriptional repressor binds to tet-O, it promotes heterochromatinization of the centromere, whereby the artificial chromosome can be removed from inside the cell. In certain embodiments, the centromere may be, for example, alpha satellite DNA (or type I alphoid DNA, e.g., type I alphoid DNA of chromosome 21 (alpha21-I)). By producing a transcriptional repressor from a gene operably linked to an inducible promoter, an artificial chromosome can be conditionally inactivated.

Destruction or removal can be done by a site-specific recombinase or a sequence-specific DNA cleavage enzyme. By operably linking a site-specific recombinase or a sequence-specific DNA cleavage enzyme to an inducible promoter, extrachromosomal DNA can be conditionally inactivated (e.g., removed from a cell). Also, by operably linking a site-specific recombinase or a sequence-specific DNA cleavage enzyme to a tissue-specific promoter, extrachromosomal DNA can be inactivated (e.g., removed from a cell) only in specific tissues. Extrachromosomal DNA can also be inactivated (e.g., removed from a cell) by removing a drug corresponding to a selection marker gene (particularly a drug resistance gene) from a culture medium, but this method has a disadvantage that inactivation (e.g., removal from a cell) may be incomplete.

When it is not necessary to preserve extrachromosomal DNA inside a cell, the extrachromosomal DNA itself can be removed from inside the cell, thereby inactivating the cassette, but when it is necessary to preserve extrachromosomal DNA inside a cell, it is preferable to remove only the cassette without removing other remaining parts from the extrachromosomal DNA. In extrachromosomal DNA preserved in a cell, other genes etc. that were not removed can exert functions in the cell even after cassette removal.

For Sendai virus, techniques for removing it from inside cells based on temperature sensitivity, techniques for removing it from inside cells by adding a tag containing a degron to a component, and techniques for removing it from inside cells by incorporating a target sequence of microRNA are known, and such techniques can also be used in the method of the present disclosure.

The cell contains at least one such cassette. It is sufficient that one cassette is inserted on DNA, and it is not necessary that two or more, for example, are inserted into each allele of the same locus. However, in certain embodiments, cassettes may be inserted into each allele of the same locus on the genome. The cell may contain two or more different cassettes integrated so as to be inactivatable (e.g., removable from cell), although not particularly limited. Two or more different cassettes can be inactivated (e.g., removed from cell) independently (e.g., at different timings).

The size of the cassette is not particularly limited, but for example, when removing the cassette using a site-specific recombinase, it may be 500 bp to 1 Mbp, for example, 1 kbp to 1 Mbp, 1 kbp to 900 kbp, 1 kbp to 800 kbp, 1 kbp to 700 kbp, 1 kbp to 600 kbp, 1 kbp to 500 kbp, 1 kbp to 400 kbp, 1 kbp to 300 kbp, 1 kbp to 200 kbp, 1 kbp to 100 kbp, 1 kbp to 50 kbp, 1 kbp to 40 kbp, 1 kbp to 30 kbp, 1 kbp to 20 kbp, 1 kbp to 10 kbp, 1 kbp to 9 kbp, 1 kbp to 8 kbp, 1 kbp to 7 kbp, 1 kbp to 6 kbp, 5 kbp, 1 kbp to 4 kbp, 1 kbp to 3 kbp, or 1 kbp to 2 kbp. The size of the cassette is not particularly limited, but when removing the cassette using a sequence-specific DNA cleavage enzyme, it may be 500 bp to 100 Mbp, 500 bp to 90 Mbp, 500 bp to 80 Mbp, 500 bp to 70 Mbp, 500 bp to 60 Mbp, 500 bp to 50 Mbp, 500 bp to 40 Mbp, 500 bp to 30 Mbp, 500 bp to 20 Mbp, 500 bp to 10 Mbp, 500 bp to 9 Mbp, 500 bp to 8 Mbp, 500 bp to 7 Mbp, 500 bp to 6 Mbp, 500 bp to 5 Mbp, 500 bp to 4 Mbp, 500 bp to 3 Mbp, 500 bp to 2 Mbp, 500 bp to 1 Mbp, 1 kbp to 900 kbp, 1 kbp to 800 kbp, 1 kbp to 700 kbp, 1 kbp to 600 kbp, 1 kbp to 500 kbp, 1 kbp to 400 kbp, 1 kbp to 300 kbp, 1 kbp to 200 kbp, 1 kbp to 100 kbp, 1 kbp to 50 kbp, 1 kbp to 40 kbp, 1 kbp to 30 kbp, 1 kbp to 20 kbp, 1 kbp to 10 kbp, 1 kbp to 9 kbp, 1 kbp to 8 kbp, 1 kbp to 7 kbp, 1 kbp to 6 kbp, 5 kbp, 1 kbp to 4 kbp, 1 kbp to 3 kbp, or 1 kbp to 2 kbp. If both ends of a cassette are respectively cleaved by a sequence-specific DNA cleavage enzyme, the cassette can be removed regardless of its size. Using a sequence-specific DNA cleavage enzyme is generally more suitable for removing a large region than using a site-specific recombinase, but those skilled in the art can appropriately select a site-specific recombinase and a sequence-specific DNA cleavage enzyme according to the size of the cassette desired to be removed. Also, those skilled in the art can appropriately determine the size of the cassette according to the site-specific recombinase and sequence-specific DNA cleavage enzyme used. The removed cassette is considered to disappear from inside the cell over time, but the removed cassette may be further cleaved or degraded to be inactivated.

In the second aspect, inactivation (e.g., removal) of the cassette is useful in evaluating functions of genes that were destroyed endogenous genes and had been complemented by exogenous genes. Inactivation (e.g., removal) of the cassette can also be performed conditionally, and by inactivating (e.g., removing) the cassette at a necessary time, it is also useful in inducing a phenotype due to destruction of an endogenous gene at the timing of inactivating (e.g., removing) the cassette. It can also be useful in tissue-specific or organ-specific inactivation (e.g., removal) like general conditional knockout.

Tissue-specific or organ-specific inactivation (e.g., removal) of a cassette can be performed by linking genes encoding a site-specific recombinase and a sequence-specific DNA cleavage enzyme to a tissue-specific or organ-specific control sequence.

The endogenous gene to be destroyed is not particularly limited and may be any gene. This is because even if it is a gene essential for cell survival, cell survival is maintained by the exogenous gene as long as it is complemented by the exogenous gene in the cassette. The endogenous gene to be destroyed may be a gene whose function is unknown. This is because the system of the present disclosure can also be used for functional analysis of genes.

The endogenous gene to be destroyed is not particularly limited, but includes, for example, genes having a physiological function, and may be selected from the group consisting of, for example, a gene related to or involved in survival of the cell, a gene related to or involved in proliferation of the cell, a gene related to or involved in differentiation control of the cell, and a gene related to or involved in a physiological function of the cell. Here, differentiation control of the cell may be maintenance of undifferentiated state (e.g., stemness or pluripotency) of the cell or promotion of differentiation of the cell. The endogenous gene to be destroyed also includes, but is not particularly limited to, for example, a gene related to or involved in or essential for cell survival or a candidate gene thereof, a gene related to or involved in or essential for cell proliferation or a candidate gene thereof, a gene related to or involved in or essential for cell differentiation or a candidate gene thereof, a gene related to or involved in or essential for cell stemness maintenance or a candidate gene thereof, a gene related to or involved in or essential for cell reprogramming or a candidate gene thereof, a gene related to or involved in cell metabolism or a candidate gene thereof, a gene encoding an enzyme of a metabolic pathway of the cell or a candidate gene thereof, a gene related to or involved in cell anabolism or a candidate gene thereof, a gene encoding an enzyme of an anabolic pathway of the cell or a candidate gene thereof, a gene related to or involved in cell motility or a candidate gene thereof, a gene encoding a cytoskeleton of the cell or a candidate gene thereof, a gene encoding a transcription factor or a candidate gene thereof, a gene encoding a transcriptional repressor or a candidate gene thereof, a gene encoding non-coding RNA (e.g., miRNA) or a candidate gene thereof, and the like. Destruction of an endogenous gene can be performed in all alleles where the gene is present. In certain embodiments, destruction of endogenous genes in some alleles rather than all alleles suffices. For example, it is beneficial when testing whether an endogenous gene causes haploinsufficiency, and when an endogenous gene causes haploinsufficiency, etc.

In certain embodiments, in the cell, the endogenous gene is not destroyed, is destroyed but not completely destroyed, or is completely destroyed. Being completely destroyed means that every single gene function is destroyed, such as complete deletion of a specific gene. Being destroyed but not completely destroyed means that at least one function of a specific gene is destroyed, but it has not reached the loss of all gene functions. By introduction of an exogenous gene, a part or more of destruction of an endogenous gene can be complemented, and by removal of a cassette containing an exogenous gene, it is possible to observe the influence of the cell changing from a complemented state to a uncomplemented state, or to obtain the benefit of the influence.

The exogenous gene that complements destruction of the endogenous gene is usually the same gene as the endogenous gene. By configuring in this way, a cassette containing an exogenous gene can be conditionally inactivated (e.g., removed) to create a situation where an endogenous gene is destroyed, so that conditional knockout of n or more genes becomes possible. However, as long as it can functionally complement destruction of the endogenous gene, the exogenous gene usually does not need to be identical to the endogenous gene.

In certain embodiments, provided is a genetically modified cell, having a cassette containing n or more exogenous genes encoding a physiologically functional product on a genome, wherein the exogenous genes are operably linked to one or more control sequences, and the cell is capable of expressing each of the exogenous genes, wherein endogenous genes corresponding to a part or all of the n or more exogenous genes are destroyed, and a part or preferably all of the destroyed endogenous genes are complemented by the exogenous genes, wherein the cassette is located between two target sequences of a sequence-specific DNA cleavage enzyme or two recognition sequences of a site-specific recombinase, and is configured such that the cassette can be removed by allowing a sequence-specific DNA cleavage enzyme or a site-specific recombinase to act on each of the two target sequences, {in the above, n is a natural number of 1 or more.}. In certain preferred embodiments, the endogenous gene and the exogenous gene are the same gene.

In certain embodiments, provided is a genetically modified cell, having a cassette containing n or more exogenous genes encoding a physiologically functional product on a genome, wherein the exogenous genes are operably linked to one or more control sequences, and the cell is capable of expressing each of the exogenous genes, wherein endogenous genes corresponding to a part or all of the n or more exogenous genes are completely destroyed (preferably deleted), and a part or preferably all of the destroyed endogenous genes are complemented by the exogenous genes, wherein the cassette is located between two target sequences of a sequence-specific DNA cleavage enzyme or two recognition sequences of a site-specific recombinase, and is configured such that the cassette can be removed by allowing a sequence-specific DNA cleavage enzyme or a site-specific recombinase to act on each of the two target sequences, {in the above, n is a natural number of 1 or more.}. In certain preferred embodiments, the endogenous gene and the exogenous gene are the same gene.

In certain embodiments, provided is a genetically modified cell, having extrachromosomal DNA, having a cassette containing n or more exogenous genes encoding a physiologically functional product on the extrachromosomal DNA, wherein the exogenous genes are operably linked to one or more control sequences, and the cell is capable of expressing each of the exogenous genes, wherein endogenous genes corresponding to a part or all of the n or more exogenous genes are destroyed, and a part or preferably all of the destroyed endogenous genes are complemented by the exogenous genes, wherein the cassette is located between two target sequences of a sequence-specific DNA cleavage enzyme or two recognition sequences of a site-specific recombinase, and is configured such that the cassette can be removed by allowing a sequence-specific DNA cleavage enzyme or a site-specific recombinase to act on each of the two target sequences, {in the above, n is a natural number of 1 or more. }. In certain preferred embodiments, the endogenous gene and the exogenous gene are the same gene.

In certain embodiments, provided is a genetically modified cell, having a cassette containing n or more exogenous genes encoding a physiologically functional product on extrachromosomal DNA, wherein the exogenous genes are operably linked to one or more control sequences, and the cell is capable of expressing each of the exogenous genes, wherein endogenous genes corresponding to a part or all of the n or more exogenous genes are completely destroyed (preferably deleted), and a part or preferably all of the destroyed endogenous genes are complemented by the exogenous genes, wherein the cassette is located between two target sequences of a sequence-specific DNA cleavage enzyme or two recognition sequences of a site-specific recombinase, and is configured such that the cassette can be removed by allowing a sequence-specific DNA cleavage enzyme or a site-specific recombinase to act on each of the two target sequences, {in the above, n is a natural number of 1 or more.}. In certain preferred embodiments, the endogenous gene and the exogenous gene are the same gene.

In certain embodiments, provided is a genetically modified cell, having extrachromosomal DNA, having a cassette containing n or more exogenous genes encoding a physiologically functional product on the extrachromosomal DNA, wherein the exogenous genes are operably linked to one or more control sequences, and the cell is capable of expressing each of the exogenous genes, wherein endogenous genes corresponding to a part or all of the n or more exogenous genes are destroyed, and a part or preferably all of the destroyed endogenous genes are complemented by the exogenous genes, wherein the extrachromosomal DNA is configured to be removable from the cell, {in the above, n is a natural number of 1 or more.}. In certain preferred embodiments, the endogenous gene and the exogenous gene are the same gene.

In certain embodiments, provided is a genetically modified cell, having a cassette containing n or more exogenous genes encoding a physiologically functional product on extrachromosomal DNA, wherein the exogenous genes are operably linked to one or more control sequences, and the cell is capable of expressing each of the exogenous genes, wherein endogenous genes corresponding to a part or all of the n or more exogenous genes are completely destroyed (preferably deleted), and a part or preferably all of the destroyed endogenous genes are complemented by the exogenous genes, wherein the extrachromosomal DNA is configured to be removable from the cell, {in the above, n is a natural number of 1 or more.}. In certain preferred embodiments, the endogenous gene and the exogenous gene are the same gene.

Inactivation (e.g., removal) of a cassette from a nucleic acid (e.g., genome) possessed by a cell can be performed based on conventional methods. For example, recognition sequences of a site-specific recombinase can be respectively arranged at both ends of the cassette, and by reacting the site-specific recombinase with the recognition sequences, the cassette can be inactivated (e.g., removed) from the nucleic acid. Also, by using DNA cleavage enzymes targeting both ends of the cassette respectively (e.g., sequence-specific DNA cleavage enzymes such as CRISPR/Cas9 system, TALEN, zinc finger nuclease, meganuclease; or site-specific recombinases such as Cre, Flp, Dre, phage integrase, transposase) to introduce cleavage specifically at both ends of the cassette, the cassette can be inactivated (e.g., removed from the nucleic acid). By arranging unique sequences recognized by the DNA cleavage enzyme at both ends of the cassette, it becomes possible to introduce cleavage specifically at both ends of the cassette.

The cell may have a gene encoding a site-specific recombinase operably linked to a control sequence (preferably, an inducible control sequence), and/or a gene encoding a sequence-specific DNA cleavage enzyme, in the cell or in DNA.

The relationship between the control sequence and the exogenous gene in the cassette will be described. The control sequence is operably linked to the exogenous gene so as to drive the exogenous gene in the cassette. In certain embodiments, the control sequence is contained within the cassette. In certain embodiments, the control sequence is not contained within the cassette but is contained in a region outside the cassette. When the control sequence is contained within the cassette, one control sequence may be operably linked to the exogenous gene so as to drive only one exogenous gene. When the control sequence is contained within the cassette, and when it is not contained within the cassette but is contained in a region outside the cassette, one control sequence may be operably linked to these exogenous genes so as to drive two or more exogenous genes. In certain embodiments, two or more exogenous genes are transcribed monocistronically. In certain embodiments, two or more exogenous genes may be configured to be transcribed polycistronically as a connected RNA. When the exogenous gene encodes a protein, the RNA may have an internal ribosome entry site (IRES) between genes. When the exogenous gene encodes a protein, two or more exogenous genes may be linked by a cleavable peptide. Examples of cleavable peptides include 2A self-cleaving peptides, cleavage sites such as furin, and the like. Examples of 2A self-cleaving peptides (2A peptides) include, but are not particularly limited to, T2A sequence, P2A sequence, E2A sequence, and F2A sequence. These sequences are well known to those skilled in the art, and those skilled in the art can appropriately obtain them.

In certain embodiments, the control sequence is not contained within the cassette but is contained in a region outside the cassette (e.g., see FIG. 2). By doing so, it is considered that a cassette fragment containing an exogenous gene generated after cassette removal is physically separated from the control sequence, and gene expression from the exogenous gene is easily stopped.

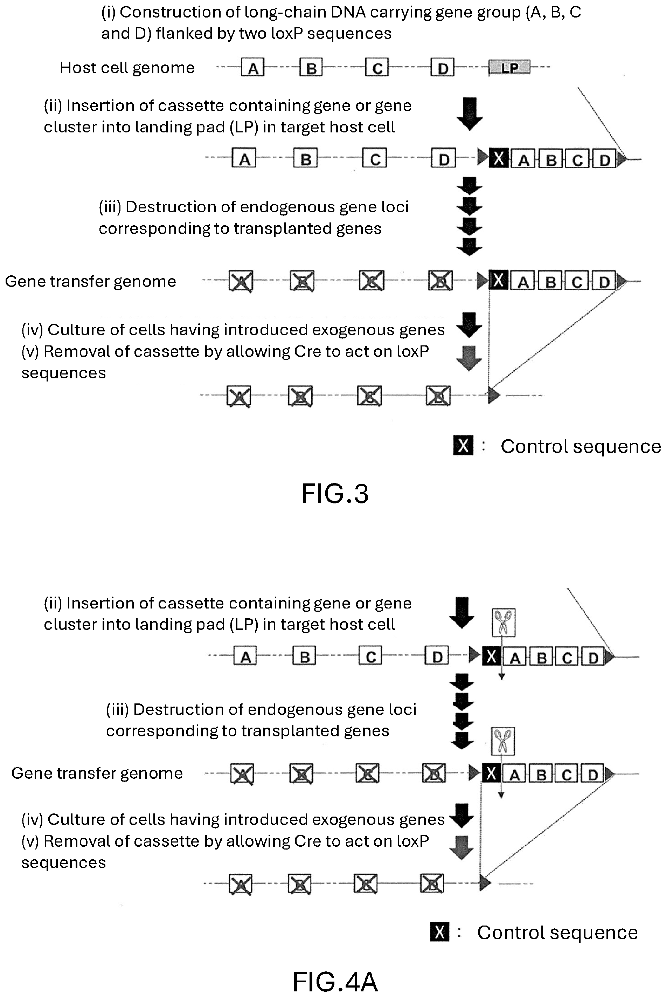

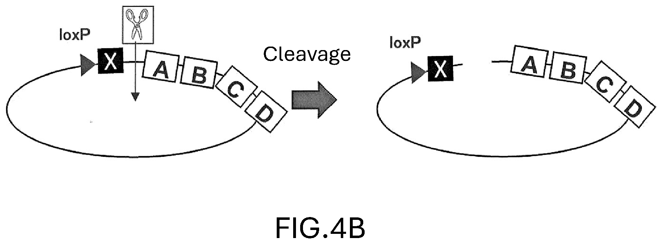

In certain embodiments, the control sequence is contained within the cassette (see FIG. 4A). Even in this case, it is considered that a cassette fragment containing an exogenous gene generated after cassette removal does not have an origin of replication etc. and is lost over time. However, if it is desired to destroy the cassette fragment, a target sequence of a sequence-specific DNA cleavage enzyme can be incorporated inside the cassette, and the target sequence can be cleaved after cassette removal (see FIG. 4B). For example, as shown in FIG. 4B, a target sequence of a sequence-specific DNA cleavage enzyme can be arranged between the control sequence and the gene in the cassette, and the control sequence and the gene can be separated by cleavage. Therefore, in certain embodiments, the control sequence is contained within the cassette, and the cassette can have a target sequence of a sequence-specific DNA cleavage enzyme inside.

According to the present disclosure, a composition comprising a cell population comprising the cell of the present disclosure is provided. In certain embodiments, the cell population comprising the cell of the present disclosure may be a cell population consisting of the cell of the present disclosure. In certain embodiments, the cell population comprising the cell of the present disclosure comprises cloned cells of the present disclosure. In certain embodiments, the cell population comprising the cell of the present disclosure comprises isolated cells of the present disclosure.

According to the present disclosure (e.g., second aspect), a gene knockout system comprising the above cell of the present disclosure is provided. The gene knockout system of the present disclosure may be a conditional knockout system. According to the present disclosure (e.g., third aspect), a silencing release system comprising the above cell is provided.

The gene knockout system of the present disclosure is preferably suitable for conditional inactivation (e.g., conditional knockout) of two or more genes, but is also suitable for conditional inactivation (e.g., conditional knockout) of one gene. For example, one gene on the genome can be moved to another location and then destroyed. For example, by introducing a negative selection marker together into the cassette, a knockout cell can be specified or determined using deletion of the negative selection marker accompanying deletion of the cassette as an indicator. While a selection marker indicates that a cell selecting the selection marker is one that has been successfully treated, a negative selection marker indicates that the presence of the marker means that treatment has failed. Negative selection markers include, for example, suicide genes (e.g., thymidine kinase such as thymidine kinase of herpes simplex virus (HSV-TK), and inducible caspase 9, etc.), fluorescent protein genes (e.g., green fluorescent protein (GFP) gene, yellow fluorescent protein (YFP) gene, red fluorescent protein (RFP) gene, etc.), luminescent enzyme genes (e.g., luciferase gene), chromogenic enzyme genes (e.g., beta-galactosidase gene, beta-glucuronidase gene, alkaline phosphatase gene, etc.), and the like. When the negative selection marker gene is a gene that has a negative effect on cell survival (e.g., suicide gene), the negative selection marker gene can be functionally linked to an inducible promoter. By functionally linking to an inducible promoter, the negative selection marker gene can be expressed only when it is desired to remove cells having the negative selection marker gene. When the negative selection marker gene has little negative effect on cell survival, such as when it is an optically detectable marker gene such as fluorescence, luminescence, and color development (visible marker gene), it may be expressed constitutively. The above mechanism of conditional knockout of one gene can be beneficial both in vivo and in vitro. For example, in vivo, it can be determined from which cell in a cell population such as tissue and organ the cassette has been removed, using deletion of a negative selection marker (e.g., a visualization marker, particularly a fluorescent dye, a chromogenic dye, etc. can be used) as an indicator. In vitro, by using a suicide gene or a toxin etc. as a negative selection marker, cells from which the cassette was not removed can be killed, and only cells from which the cassette was removed can be concentrated. Also, it is advantageous in that conditional knockout can be performed without being affected by unforeseen effects due to the native genomic location.

The conditional gene inactivation system (e.g., conditional knockout system) of the present disclosure may also be beneficial in that a plurality of genes scattered on the genome can be collectively conditionally inactivated (e.g., knocked out). By doing so, a useful inactivation (e.g., knockout) system is provided in which a plurality of genes can be collectively knocked out at the timing of removing the cassette.

### <Method for Manufacturing Cell of the Present Disclosure>

The cell of the present disclosure can be manufactured, for example, as described in Examples described later, although not particularly limited thereto. The cell of the present disclosure can also be manufactured by methods detailed below.

According to the present disclosure, a method for manufacturing the cell of the present disclosure is provided. The method comprises providing a cell (starting cell). The starting cell may be, for example, a cell having an endogenous gene scheduled to be subjected to destruction.

The starting cell may be the cell described above.

In certain embodiments, the starting cell may be one that does not have a destroyed endogenous gene.

In certain embodiments, in the starting cell, one or more endogenous genes may be destroyed as long as implementation of the present disclosure is not significantly hindered, and preferably, the cell can survive or proliferate in culture under appropriate conditions. These endogenous genes may be destroyed but not completely destroyed, or completely destroyed. Being completely destroyed means that every single gene function is destroyed, such as complete deletion of a gene. Being destroyed but not completely destroyed means that at least one gene function is destroyed, but it has not reached the loss of all gene functions. For example, it will be understood that even if the starting cell already had a destroyed endogenous gene, it is acceptable as long as the destruction does not inhibit subsequent cell modification steps. In subsequent modification steps, it is not necessary to complement this endogenous gene with an exogenous gene, but when complementing with an exogenous gene, it can also be used to test the function of the exogenous gene.

The method also comprises inserting a cassette containing n or more exogenous genes into a cell (e.g., into a transcriptionally active locus of the cell). According to the present disclosure, a cell having such a cassette, and a method for manufacturing the cell are provided.

In the second aspect and the fourth aspect, the method further comprises destroying the endogenous gene. The number of endogenous genes to be destroyed is 1 or more, preferably 2 or more. Even by destruction of an endogenous gene, since its function is complemented by an exogenous gene supplied to the cassette, influence of destruction of the endogenous gene on the cell may occur, but may be limited. In order for the function to be complemented by the exogenous gene, it is preferable that the endogenous gene and the exogenous gene are the same gene, but they do not need to have completely identical sequences to each other. According to the present disclosure, provided is a method for simultaneously inactivating (e.g., knocking out) 1 or more, preferably 2 or more genes. The method comprises first providing a cell having a cassette as described above.

In the second aspect, the third aspect, and the fourth aspect, the method further comprises inactivating (e.g., removing) the cassette. Thereby, n or more exogenous genes loaded in the cassette can be inactivated (e.g., removed) from inside the cell at once, and n or more exogenous genes can be collectively inactivated. Therefore, in the second aspect and the fourth aspect, by utilizing this method, the effect of destruction of the endogenous gene can be significantly generated by inactivation (e.g., removal) of the cassette, not by the timing of destruction of the endogenous gene, whereby conditional inactivation (e.g., knockout) of the endogenous gene (preferably simultaneous conditional inactivation (e.g., knockout) of 2 or more genes) becomes possible. In the method of the present disclosure, if the method is designed so that the entire cassette can be inactivated (e.g., removed), all genes contained in the cassette are collectively inactivated (e.g., removed), and more complete simultaneous conditional inactivation (e.g., knockout) of multiple genes is achieved. Also, in the third aspect and the fourth aspect, by removing the cassette, the knockdown nucleic acid supplied to the cassette is inactivated (e.g., removed), and its silencing effect can be stopped.

In manufacture of the cell of the present disclosure, as described later, a cell (intermediate cell) into which a landing pad (LP; exogenous target region, e.g., see FIG. 1(i)) enabling introduction of an exogenous gene into one region on the genome of the cell or onto DNA outside the host chromosome is inserted can be produced. The landing pad functions as a foundation for integrating the cassette, and may contain various factors other than the cassette. This intermediate cell is suitable for manufacture of cells of any of the first aspect to the fourth aspect.

For example, the landing pad may contain a control sequence.

For example, the landing pad may contain one recognition sequence of a site-specific recombinase or target sequence of a sequence-specific DNA cleavage enzyme on each of the outsides of the site where the cassette is inserted (i.e., a total of two).

For example, the landing pad may contain insulator sequences respectively near both ends thereof. An insulator sequence refers to a sequence that blocks or mitigates the influence of adjacent chromosomal environments and guarantees or enhances independence of transcriptional regulation of DNA sandwiched between the regions. Insulators are defined by enhancer blocking effect (effect of blocking influence of an enhancer on promoter activity by inserting between the enhancer and a promoter), and position effect suppressing action (action of preventing expression of a transgene from being affected by the inserted position by sandwiching both sides of the transgene with insulators).

The relationship between the control sequence and the cassette is as described above.

In certain embodiments, as shown in FIG. 1C, two recognition sequences of a site-specific recombinase or target sequences of a sequence-specific DNA cleavage enzyme are contained, and a region for inserting a cassette can be contained in a region between the two recognition sequences and target sequences. In certain embodiments, insulator sequences may be arranged before and after, respectively, so as to sandwich two recognition sequences of a site-specific recombinase or target sequences of a sequence-specific DNA cleavage enzyme. In certain embodiments, the landing pad may contain one or more control sequences in the vicinity of the region for inserting the cassette so as to be operably linked to the exogenous gene in the cassette.

In certain embodiments, the region for inserting the cassette in the landing pad can contain a target sequence of a sequence-specific DNA cleavage enzyme. This is because the cassette can be inserted at the location of the target sequence by cleaving the target sequence in the presence of donor DNA containing the cassette. Here, the donor DNA has an upstream homology arm capable of homologous recombination with an upstream region of the target sequence in the landing pad, and a downstream homology arm capable of homologous recombination with downstream of the target sequence, and has a cassette in a region between the upstream homology arm and the downstream homology arm. By using such donor DNA, the region between the upstream homology arm and the downstream homology arm of the donor DNA (i.e., the region having the cassette) is inserted between the upstream region and the downstream region.

In certain embodiments, as shown in FIG. 1B, the region for inserting the cassette in the landing pad does not have to contain a target sequence of a sequence-specific DNA cleavage enzyme. A region (target region) within the landing pad desired to be inserted can be assumed, and it has an upstream homology arm capable of homologous recombination with an upstream region of the target sequence in the landing pad, and a downstream homology arm capable of homologous recombination with downstream of the target sequence, and has a cassette in a region between the upstream homology arm and the downstream homology arm. By using such donor DNA, the region between the upstream homology arm and the downstream homology arm of the donor DNA (i.e., the region having the cassette) is inserted between the upstream region and the downstream region.

Of course, the cell of the present disclosure can be manufactured without using a landing pad. Donor DNA can be integrated into DNA, for example, by the method disclosed in WO 2021/206054. An advantage of using a landing pad is that by supplying components common to modified cells onto the landing pad, components included in donor DNA for insertion into modified cells can be reduced. Or, by introducing a unique cleavable sequence (target sequence, e.g., recognition sequence of meganuclease, target sequence of genome editing enzyme) into DNA (particularly, genome) on the landing pad, risk of genome cleavage at another location due to off-target during exogenous gene introduction can be reduced.

The cassette can be produced by ordinary gene recombination techniques or DNA synthesis techniques. For example, techniques for synthesizing DNA encoding genes carrying cassettes respectively, making ends of DNA sticky ends, and ligating them with other DNA, etc., to ligate a plurality of DNA fragments to obtain a single DNA fragment are known and widely used. By utilizing such techniques, a cassette loaded with n or more genes can be easily obtained.

### <Method for Culturing Cell of the Present Disclosure>

According to the present disclosure, a method for culturing the cell of the present disclosure is provided. This method comprises providing the cell of the present disclosure (having a cassette containing n or more exogenous genes). The method also comprises culturing the cell of the present disclosure under conditions suitable for culture, allowing a site-specific recombinase to act on recognition sequences of the site-specific recombinase sandwiching the cassette to inactivate the cassette (e.g., delete from DNA (e.g., remove from genome or extrachromosomal DNA)), and thereafter further culturing the cell. The site-specific recombinase for the recognition sequences of the site-specific recombinase may be integrated expressibly on extrachromosomal DNA, or outside or inside the cassette on the genome, and by configuring in this way, the cassette can be inactivated (e.g., removed) by inducing expression of the enzyme. Expression induction of the above enzyme can be achieved by those skilled in the art using, for example, an inducible promoter or the like. Expression induction of the above enzyme can also be achieved by those skilled in the art using, for example, a tissue-specific or organ-specific promoter or the like.

### <Examples of Use of Cell of the Present Disclosure>

Further examples of use of the cell of the present disclosure are as described in Examples described later.

### [Examples]

### Example 1: Construction of novel knockout system

A new knockout system different from conventional conditional knockout techniques is devised. The new knockout system can efficiently knockout a plurality of genes scattered on the genome by a single operation.

For construction of this knockout system, a cell into which a landing pad (LP; exogenous target region, e.g., see FIG. 1(i)) enabling introduction of an exogenous gene into one region on the genome of the cell is inserted can be produced. It is desired that the landing pad is introduced at a position that does not affect expression of existing genes on the genome. The landing pad can be inserted, for example, into a transcriptionally active locus or a safe harbor region of the genome. The cell having insertion of the landing pad obtained as above is advantageously used for construction of subsequent knockout systems. Naturally, cells having no landing pad can also be used for construction of subsequent knockout systems.

Next, a fragment (cassette) containing one or more genes to be knocked out is introduced into an arbitrary target region or landing pad on the genome (e.g., see FIG. 1(ii)). The number of genes contained in the cassette may be one, but preferably contains two or more genes. FIG. 1(ii) shows an example of insertion of a cassette containing four genes. It is preferable that genes contained in this cassette are operably linked to a control sequence and expression induction is possible for each. The reason is that even if an endogenous gene is destroyed in a later step, it is possible to rescue a phenotype due to destruction by expressing the gene contained in the cassette, or to attempt the rescue.

Furthermore, endogenous genes corresponding to genes contained in the cassette are respectively destroyed. Destruction of genes can be performed by known methods or well-known common techniques such as destruction of the coding region and destruction of the control sequence thereof. The number of endogenous genes to be destroyed is 1 or more, preferably 2 or more (e.g., see FIG. 1(iii)). Genes corresponding to endogenous genes to be destroyed are integrated into the cassette in a manner capable of inducing expression, and phenotypes due to destruction of endogenous genes can be rescued.

The cassette can be removed at a desired timing. For example, FIG. 1 shows that the cassette is located between loxP sequences, and the cassette can be removed by allowing Cre recombinase to act thereon. A plurality of genes are included in the cassette, and it results in being able to knock out these collectively. Thus, the knockout system of the present disclosure is useful when conditionally knocking out one or more genes.

### Example 2: Application Examples of Knockout System

### (1) Functional Analysis of Genes

The knockout system of the present disclosure can be used for functional analysis of genes. More specifically, since one or more genes, preferably a plurality of genes, can be conditionally knocked out, the knockout system of the present disclosure can be advantageously used when examining functions of a combination of one or more genes, preferably a plurality of genes. Further, even if the endogenous gene to be destroyed includes a gene essential for survival or proliferation of a cell, if it is before removal of the cassette, since the cell possesses a gene corresponding to the gene in an expressible manner, it is possible not to receive an influence of the endogenous gene until removal of the cassette, and by removing the cassette at a desired timing, the gene is knocked out at the desired timing, and by observing a phenotype brought about by destruction, a function that the destroyed gene possessed can be estimated. It is not a problem that an additional gene is included in the cassette in addition to the endogenous gene to be destroyed. This is because it is possible to utilize a function of the additional gene, or to investigate the function if the function is unknown. Further, genes corresponding to all of the endogenous genes to be destroyed do not necessarily need to be included in the cassette. This is because if complementation by the cassette is unnecessary, it is not necessary to introduce it into the cassette to induce gene expression.

Since a gene can be complemented by the cassette, there is no particular limitation on the endogenous gene to be destroyed, and the endogenous gene to be destroyed may include any of existing genes. Examples include a gene essential for survival of a cell or a candidate gene thereof, a gene essential for proliferation of a cell or a candidate gene thereof, a gene essential for differentiation of a cell or a candidate gene thereof, a gene essential for maintenance of stemness of a cell or a candidate gene thereof, a gene essential for reprogramming of a cell or a candidate gene thereof, a gene encoding an enzyme of a metabolic pathway of a cell or a candidate gene thereof, a gene encoding an enzyme of an anabolic pathway of a cell or a candidate gene thereof, a gene encoding a skeleton of a cell or a candidate gene thereof, a gene encoding a transcription factor or a candidate gene thereof, a gene encoding a transcription repressor or a candidate gene thereof, a gene encoding a non-coding RNA or a candidate gene thereof, and the like. The gene to be complemented in the cassette may be a gene having a known function. This is because it is possible to evaluate whether destruction of the endogenous gene can be rescued by the known function, and further there is a possibility that an unknown gene function can be elucidated.

### (2) Substance Production by Cells

A production system of a useful substance using a cell is advantageously used industrially. In some cases, by destroying an enzyme such as a metabolic enzyme, a metabolic pathway is changed, and thereby a desired substance is obtained in a large amount compared to before destruction. However, destruction of such an enzyme may bring about an adverse effect on proliferation of the cell in some cases. Therefore, in a proliferation phase of the cell (for example, a logarithmic growth phase), culture is performed in an environment where the enzyme is present, and after the cell proliferates, by removing the cassette and then allowing substance production, it can be used to increase a yield of the desired substance while preventing an influence brought about on cell proliferation. At the time of substance production, nutrients containing a sufficient carbon source can be supplied to the cell under appropriate conditions.

In substance production using a cell, a cell can be used in which a gene necessary for proliferation of the cell is destroyed and which contains a cassette having a corresponding gene. In a proliferation phase of the cell (for example, a logarithmic growth phase), culture is performed in an environment where the gene functions, and after the cell proliferates, by removing the cassette and then allowing substance production, it can be used to increase a yield of the desired substance while preventing an influence brought about on cell proliferation. In particular, the cell allocates utilizable energy to cell proliferation and substance production, but by reducing an energy amount and/or a carbon source amount distributed to cell proliferation, it can be expected to increase an energy and/or a carbon source amount utilizable for substance production. By doing so, substance production by the cell can be made efficient. At the time of substance production, nutrients containing a sufficient carbon source can be supplied to the cell under appropriate conditions. In this aspect, it is desirable that the cell survives and proliferates before removal of the cassette, and after removal of the cassette, it at least survives but does not have to proliferate. For example, in a certain aspect, after removal of the cassette, the cell has reduced its proliferation ability compared to before removal of the cassette. In a certain aspect, after removal of the cassette, the cell does not substantially show proliferation ability, and for example, does not increase the number of cells by 2 times or more, 1.5 times or more, 1.4 times or more, 1.3 times or more, 1.2 times or more, or 1.1 times or more.

As a more specific example, the following can be exemplified. In this specific example, the cell is a bacterium (preferably a hydrogen bacterium). The bacterium has a cassette, and exogenous genes having a plurality of physiological functions are included in the cassette. As for the exogenous genes, one or more, particularly two or more endogenous genes selected from the group consisting of enzymes of a peptidoglycan synthesis pathway (for example, glmS and glmU), enzymes of a fatty acid synthesis pathway (for example, fabH and fabD), leucine synthases (for example, leuA1 and leuA2), enzymes of a pathway from acetyl-CoA to the TCA cycle (for example, cisY), and hbdH are destroyed. Further, the exogenous gene can complement the endogenous gene to be destroyed, and preferably includes exogenous genes corresponding to all of the endogenous genes to be destroyed among at least the endogenous genes. Such bacteria can be proliferated under conditions suitable for culture in the presence of the cassette. When the bacteria have proliferated sufficiently, the cassette can be inactivated (for example, removed). It is considered that by further culturing the bacteria after removal of the cassette, the bacteria come to synthesize polyhydroxyalkane (PHA) efficiently. This is because it can be expected that by the enzyme being inactivated (particularly removed), production pathways other than PHA are stopped, and the carbon source and the energy source become able to be efficiently used for PHA synthesis.

### (3) Maintenance of Undifferentiated State or Induction of Differentiation of Cells

Maintenance of an undifferentiated state of a cell is important from the viewpoint of quality control of stem cells (particularly, pluripotent cells or pluripotent stem cells). On the other hand, when maintenance of the undifferentiated state is no longer necessary, it may be desired to turn off gene expression for maintenance of the undifferentiated state. In such a case, by destroying an endogenous gene of the cell in advance and complementing a corresponding gene on the cassette, the undifferentiated state is maintained by the complemented gene function, and when maintenance of the undifferentiated state becomes unnecessary, the cassette can be removed. As the time when maintenance of the undifferentiated state is not necessary, for example, a time when inducing differentiation of the cell can be mentioned. As a gene for maintaining the cell in the undifferentiated state, Oct4 can be mentioned. As genes for maintaining the cell in the undifferentiated state, klf4, c-mic, sox2, nanog, lin28, and the like can also be mentioned. Therefore, by introducing one or more of these into the cassette and making them expressible, and thereupon destroying one or more of the endogenous genes, a cell can be obtained which is useful for maintenance of the undifferentiated state, while being able to promote induction of differentiation by inactivating (for example, removing) the cassette when it is desired to differentiate. In particular, sox2 plays an important role in tissue differentiation into ectoderm together with maintenance of pluripotency of pluripotent stem cells. And, it is considered that by inactivating (for example, removing) sox2, differentiation of the cell into endomesoderm is promoted together with loss of pluripotency. This technique is considered to be useful, for example, when differentiating pancreatic islets or pancreatic beta cells from pluripotent stem cells.

Maintenance of the undifferentiated state is not limited to pluripotent stem cells, and may also be useful for maintenance of other types of tissue stem cells and progenitor cells.

Further, it may also be useful in induction of differentiation of cells. Although not particularly limited, this is because there is a case where a gene necessary in one step of a differentiation induction process of a cell becomes unnecessary in a subsequent differentiation step. Although not particularly limited, in such a case, after destroying a corresponding endogenous gene, a corresponding gene is complemented in the cassette, and when one differentiation step is completed, the next differentiation step can be performed by removing the cassette. By removing the cassette and the gene inside thereof, an effect of preventing unexpected induction of differentiation of the cell due to expression of the gene can be expected. When a certain cell can differentiate into either of cell A and B, differentiation into cell A can be promoted by removing a gene promoting differentiation into cell B. Further, it is considered that differentiation into cell A can be further promoted by introducing a knockdown nucleic acid against a gene promoting differentiation into cell A into the cassette, and removing the cassette when promoting differentiation into cell A.

## Claims

1. A genetically modified cell, comprising a cassette containing n or more exogenous genes encoding a physiologically functional product, wherein the exogenous gene is operably linked to one or more control sequences, the cell can express each of the exogenous genes, an endogenous gene corresponding to a part or all of the n or more exogenous genes is destroyed, and the cassette is configured to be able to be conditionally inactivated {wherein n is a natural number of 1 or more}.

2. The cell according to claim 1, wherein n is a natural number of 2 or more.

3. The cell according to claim 1 or 2, wherein the destroyed endogenous gene includes a gene related to or involved in survival or proliferation of the cell, or a candidate gene thereof.

4. The cell according to claim 1 or 2, wherein the destroyed endogenous gene includes a gene related to or involved in differentiation control of the cell, or a candidate gene thereof.

5. The cell according to claim 1 or 2, wherein the destroyed endogenous gene includes a gene related to or involved in a physiological function of the cell, or a candidate gene thereof.

6. The cell according to any one of claims 1 to 5, wherein the control sequence is located outside a region between the two target sequences or the two recognition sequences (including the sequences).

7. The cell according to any one of claims 1 to 6, wherein the cassette further carries a gene encoding a knockdown nucleic acid.

8. A composition for use in producing the cell according to any one of claims 1 to 7, comprising a cell having an exogenous target region for incorporating the cassette, wherein the exogenous target region includes a target sequence of a sequence-specific DNA cleavage enzyme.

9. The composition according to claim 8, wherein the exogenous target region includes two alleles, each allele has a positive selection marker that is distinguishably different from each other, and each allele further has a negative selection marker, provided that, when the positive selection marker also serves as the negative selection marker, the allele is not required to have a negative marker.

10. A method, comprising:
(A) culturing the cell according to any one of claims 1 to 7 under appropriate conditions;
(B) inactivating the cassette after culture to obtain a cell in which the cassette is inactivated; and
(C) further culturing the cell in which the cassette is inactivated under appropriate conditions.

11. The method according to claim 10, wherein the destroyed gene is a gene related to or involved in survival or proliferation of the cell or a candidate gene thereof, and the step (B) is performed after proliferating the cell in the step (A).

12. The method according to claim 10, wherein the destroyed gene is a gene related to or involved in differentiation control of the cell or a candidate gene thereof, and the step (B) is performed after differentiating the cell in the step (A).

13. The method according to claim 10, wherein the destroyed gene is a gene related to or involved in differentiation control of the cell or a candidate gene thereof, and comprising subjecting the cell to differentiation treatment in the culture of the step (C) after performing the step (B).

14. The method according to claim 10, wherein the destroyed gene is a gene related to or involved in a physiological function of the cell or a candidate gene thereof, and the step (B) is performed after proliferating the cell in the step (A).
